# EUROPÄISCHE PATENTSCHRIFT

(11) **EP 1 809 629 B1**
(45) Veröffentlichungstag und Bekanntmachung des Hinweises auf die Patenterteilung: **13.06.2012**
(21) Anmeldenummer: 05791308.9
(22) Anmeldetag: 12.10.2005
(51) Int. Cl.: C07D 471/04, A61K 31/519, A61P 35/00

(54) **Zur Behandlungen von Tumoren, Augenkrankheiten, Entzündungen sowie Erkrankungen des Immunsystems geeignete Derivate des N,N'-Diphenylharnstoffs**
N,N'-diphenyl urea derivatives suitable for the treatment of tumours, eye diseases, inflammation and diseases of the immune system
Dérivés de N,N'-diphenylurée utiles pour le traitement de tumeurs, de maladies oculaires, d'inflammation et de maladies immunitaires

(30) Priorität: 10.11.2004 DE 102004054216
(43) Veröffentlichungstag der Anmeldung: 25.07.2007
(73) Patentinhaber: Merck Patent GmbH, 64293 Darmstadt (DE)
(72) Erfinder: HOELZEMANN, Guenter, 64342 Seeheim-Jugenheim (DE); CRASSIER, Helene, 64291 Darmstadt (DE); JONCZYK, Alfred, 64295 Darmstadt (DE); RAUTENBERG, Wilfried, 64354 Reinheim (DE)
(86) Internationale Anmeldenummer: PCT/EP2005/010957
(87) Internationale Veröffentlichungsnummer: WO 2006/050779

(56) Entgegenhaltungen:
- WO-A-2004/078747
- J.-L. GIRARDET ET AL.: "Synthesis and Cytotoxixity of 4-Amino-5-oxopyrido[2,3-d]pyrimidine Nucleosides" JOURNAL OF MEDICINAL CHEMISTRY., Nr. 43, 2000, Seiten 3704-3713, XP002367886 AMERICAN CHEMICAL SOCIETY. WASHINGTON; US

## Beschreibung

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verwendung von Verbindungen, bei denen die Hemmung, Regulierung und/oder Modulation der Signaltransduktion von Kinasen, insbesondere der Tyrosinkinasen und/oder Serin/Threonin-Kinasen eine Rolle spielt, ferner pharmazeutische Zusammensetzungen, die diese Verbindungen enthalten, sowie die Verwendung der Verbindungen zur Behandlung kinasebedingter Krankheiten.

Im einzelnen betrifft die vorliegende Erfindung Verbindungen der Formel I, die die Signaltransduktion der Tyrosinkinasen hemmen, regulieren und/oder modulieren, Zusammensetzungen, die diese Verbindungen enthalten, sowie Verfahren zu ihrer Verwendung zur Behandlung von tyrosinkinasebedingten Krankheiten und Leiden wie Angiogenese, Krebs, Tumorentstehung, -wachstum und -verbreitung, Arteriosklerose, Augenerkrankungen, wie altersbedingte Makula-Degeneration, choroidale Neovaskularisierung und diabetische Retinopathie, Entzündungserkrankungen, Arthritis, Thrombose, Fibrose, Glomerulonephritis, Neurodegeneration, Psoriasis, Restenose, Wundheilung, Transplantatabstossung, metabolische und Erkrankungen des Immunsystems, auch Autoimmunerkrankungen, Zirrhose, Diabetes und Erkrankungen der Blutgefässe, dabei auch Instabilität und Durchlässigkeit (Permeabilität) und dergleichen bei Säugetieren.

Bei den Tyrosinkinasen handelt es sich um eine Klasse von Enyzmen mit mindestens 400 Mitgliedern, die die Übertragung des endständigen Phosphats des Adenosintriphosphats (gamma-Phosphat) auf Tyrosinreste bei Proteinsubstraten katalysieren. Man nimmt an, dass den Tyrosinkinasen bei verschiedenen Zellfunktionen über die Substratphosphorylierung eine wesentliche Rolle bei der Signaltransduktion zukommt. Obwohl die genauen Mechanismen der Signaltransduktion noch unklar sind, wurde gezeigt, dass die Tyrosinkinasen wichtige Faktoren bei der Zellproliferation, der Karzinogenese und der Zelldifferenzierung darstellen. Die Tyrosinkinasen lassen sich in Rezeptor-Tyrosinkinasen und zytosolische Tyrosinkinasen einteilen. Die Rezeptor-Tyrosinkinasen weisen einen extrazellulären Teil, einen Transmembranteil und einen intrazellulären Teil auf, während die zytosolischen Tyrosinkinasen ausschließlich intrazellulär vorliegen. (siehe Reviews von Schlessinger und Ullrich, Neuron 9, 383-391 (1992) und 1-20 (1992)).
Die Rezeptor-Tyrosinkinasen bestehen aus einer Vielzahl von Transmembranrezeptoren mit unterschiedlicher biologischer Wirksamkeit. So wurden ungefähr 20 verschiedene Unterfamilien von Rezeptor-Tyrosinkinasen identifiziert. Eine Tyrosinkinase-Unterfamilie, die die Bezeichnung HER-Unterfamilie trägt, besteht aus EGFR, HER2, HER3 und HER4. Zu den Liganden dieser Rezeptor-Unterfamilie zählen der Epithel-Wachstumsfaktor, TGF-α, Amphiregulin, HB-EGF, Betacellulin und Heregulin. Die Insulin-Unterfamilie, zu der INS-R, IGF-IR und IR-R zählen, stellt eine weitere Unterfamilie dieser Rezeptor-Tyrosinkinasen dar. Die PDGF-Unterfamilie beinhaltet den PDGF-α- and -β-Rezeptor, CSFIR, c-kit und FLK-II. Außerdem gibt es die FLK-Familie, die aus dem Kinaseinsertdomänenrezeptor (KDR), der fötalen Leberkinase-1 (FLK-1), der fötalen Leberkinase-4 (FLK-4) und der fms-Tyrosinkinase-1 (flt-1) besteht. Die PDGF- und FLK-Familie werden üblicherweise aufgrund der zwischen den beiden Gruppen bestehenden Ähnlichkeiten gemeinsam diskutiert. Für eine genaue Diskussion der Rezeptor-Tyrosinkinasen siehe die Arbeit von Plowman et al., DN & P 7(6):334-339, 1994, die hiermit durch Bezugnahme aufgenommen wird.
Zu den RTKs (Rezeptor-Tyrosin-Kinasen) gehören auch TIE2 und seine Liganden Angiopoietin 1 und 2. Es werden mittlerweile immer mehr Homologe dieser Liganden gefunden, deren Wirkung im Einzelnen noch nicht klar nachgewiesen wurde. Als Homologes von TIE2 ist TIE1 bekannt. Die TIE RTKs werden selektiv auf Endothelzellen exprimiert und finden ihre Aufgabe bei Prozessen der Angiogenese und Maturierung der Blutgefäße. Dadurch können sie insbesondere bei Erkrankungen des Gefäßsystems und bei Pathologien, in denen Gefäße genutzt oder gar umgebildet werden, ein wertvolles Ziel sein. Ausser der Verhinderung der Gefäßneubildung und Maturierung kann auch die Stimulation von Gefäßneubildung ein wertvolles Ziel für Wirkstoffe sein. Bezug genommen wird auf Übersichtsarbeiten zur Angiogenese, Tumorentwicklung und Kinase Signalgebung von
G. Breier Placenta (2000) 21, Suppl A, Trophoblasr Res 14, S11-S15
F. Bussolino et al. TIBS 22, 251 -256 (1997)
G. Bergers & L.E. Benjamin Nature Rev Cancer 3, 401-410 (2003)
P. Blume-Jensen & . Hunter Nature 411, 355-365 (2001)
M. Ramsauer & P. D'Amore J. Clin. INvest. 110, 1615-1617 (2002)
S. Tsigkos et al. Expert Opin. Investig. Drugs 12, 933-941 (2003)

Beispiele für Kinase-Inhibitoren, die bereits in der Krebstherapie getestet werden, können L.K. Shawyer et al. Cancer Cell 1, 117-123(2002) und D. Fabbro & C. Garcia-Echeverria Current Opin. Drug Discovery & Development 5, 701-712 (2002) entnommen werden.

Die zytosolischen Tyrosinkinasen bestehen ebenfalls aus einer Vielzahl von Unterfamilien, darunter Src, Frk, Btk, Csk, Abl, Zap70, Fes/Fps, Fak, Jak, Ack, and LIMK. Jede dieser Unterfamilien ist weiter in verschiedene Rezeptoren unterteilt. So stellt zum Beispiel die Src-Unterfamilie eine der größten Unterfamilien dar. Sie beinhaltet Src, Yes, Fyn, Lyn, Lck, Blk, Hck, Fgr und Yrk. Die Src-Enzymunterfamilie wurde mit der Onkogenese in Verbindung gebracht. Für eine genauere Diskussion der zytosolischen Tyrosinkinasen, siehe die Arbeit von Bolen Oncogene, 8:2025-2031 (1993), die hiermit durch Bezugnahme aufgenommen wird.
Sowohl die Rezeptor-Tyrosinkinasen als auch die zytosolischen Tyrosinkinasen sind an Signalübertragungswegen der Zelle, die zu verschiedenen Leidenszuständen führen, darunter Krebs, Schuppenflechte und Hyperimmunreaktionen, beteiligt.
Es wurde vorgeschlagen, dass verschiedene Rezeptor-Tyrosinkinasen sowie die an sie bindenden Wachstumsfaktoren eine Rolle bei den Angiogenese spielen, obwohl einige die Angiogenese indirekt fördern könnten (Mustonen und Alitalo, J. Cell Biol. 129:895-898, 1995). Eine dieser Rezeptor-Tyrosinkinasen ist die fötale Leberkinase 1, auch FLK-1 genannt. Das menschliche Analog der FLK-1 ist der kinase-insertdomänenhaltige Rezeptor KDR, der auch unter der Bezeichnung Gefäßendothelzellenwachstumsfaktorrezeptor 2 bzw. VEGFR-2 bekannt ist, da er VEGF hochaffin bindet. Schließlich wurde die Maus-Version dieses Rezeptors auch ebenfalls NYK genannt (Oelrichs et al., Oncogene 8(1):11-15, 1993). VEGF und KDR stellen ein Ligand-Rezeptor-Paar dar, das eine wesentliche Rolle bei der Proliferation der Gefäßendothelzellen und der Bildung und Sprossung der Blutgefäße, die als Vaskulogenese bzw. Angiogenese bezeichnet werden, spielt.
Die Angiogenese ist durch eine übermäßig starke Aktivität des Gefäßendothelwachstumsfaktors (VEGF) gekennzeichnet. Der VEGF besteht eigentlich aus einer Familie von Liganden (Klagsburn und D'Amore, Cytokine & Growth Factor Reviews 7:259-270, 1996). Der VEGF bindet den hochaffinen transmembranösen Tyrosinkinaserezepzor KDR und die verwandte fms-Tyrosinkinase-1, auch unter der Bezeichnung Flt-1 oder Gefäßendothelzellenwachstumsfaktorrezeptor 1 (VEGFR-1) bekannt. Aus Zellkultur- und Gen- Knockout-Versuchen geht hervor, dass jeder Rezeptor zu unterschiedlichen Aspekten der Angiogenese beiträgt. Der KDR führt die mitogene Funktion des VEGF herbei, während Flt-1 nichtmitogene Funktionen, wie diejenigen, die mit der Zelladhäsion in Zusammenhang stehen, zu modulieren scheint. Eine Hemmung des KDR moduliert daher das Niveau der mitogenen VEGF-Aktivität. Tatsächlich wurde gezeigt, dass das Tumorwachstum von der antiangiogenen Wirkung der VEGF-Rezeptor-Antagonisten beeinflusst wird (Kim et al., Nature 362, S. 841- 844, 1993).
Drei PTK (Protein-Tyrosinkinase)-Rezeptoren für VEGFR sind identifiziert worden : VEGFR-1 (Flt-1); VEGRF-2 (Flk-1 oder KDR) und VEGFR-3 (Flt-4). Von besonderem Interesse ist VEGFR-2.

Feste Tumore können daher mit Tyrosinkinasehemmern behandelt werden, da diese Tumore für die Bildung der zur Unterstützung ihres Wachstums erforderlichen Blutgefäße auf Angiogenese angewiesen sind. Zu diesen festen Tumoren zählen die Monozytenleukämie, Hirn-, Urogenital-, Lymphsystem-, Magen-, Kehlkopf- und Lungenkarzinom, darunter Lungenadenokarzinom und kleinzelliges Lungenkarzinom. Zu weiteren Beispielen zählen Karzinome, bei denen eine Überexpression oder Aktivierung von Raf-aktivierenden Onkogenen (z.B. K-ras, erb-B) beobachtet wird. Zu diesen Karzinomen zählen Bauchspeicheldrüsen- und Brustkarzinom. Hemmstoffe dieser Tyrosinkinasen eignen sich daher zur Vorbeugung und Behandlung von proliferativen Krankheiten, die durch diese Enzyme bedingt sind.
Die angiogene Aktivität des VEGF ist nicht auf Tumore beschränkt. Der VEGF ist für die bei diabetischer Retinopathie in bzw. in der Nähe der Retina produzierte angiogene Aktivität verantwortlich. Dieses Gefäßwachstum in der Retina führt zu geschwächter Sehkraft und schließlich Erblindung. Die VEGF-mRNA- und -protein-Spiegel im Auge werden durch Leiden wie Netzhautvenenokklusion beim Primaten sowie verringertem pO₂-Spiegel bei der Maus, die zu Gefäßneubildung führen, erhöht. Intraokular injizierte monoklonale Anti-VEGF-Antikörper, oder VEGF-Rezeptor-Immunkonjugate, hemmen sowohl im Primaten- als auch im Nagetiermodell die Gefäßneubildung im Auge. Unabhängig vom Grund der Induktion des VEGF bei der diabetischen Retinopathie des Menschen, eignet sich die Hemmung des Augen-VEGF zur Behandlung dieser Krankheit.
Die VEGF-Expression ist auch in hypoxischen Regionen von tierischen und menschlichen Tumoren neben Nekrosezonen stark erhöht. Der VEGF wird außerdem durch die Expression der Onkogene ras, raf, src und p53-Mutante (die alle bei der Bekämpfung von Krebs von Bedeutung sind) hinaufreguliert. Monoklonale Anti-VEGF-Antikörper hemmen bei der Nacktmaus das Wachstum menschlicher Tumore. Obwohl die gleichen Tumorzellen in Kultur weiterhin VEGF exprimieren, verringern die Antikörper ihre Zellteilungsrate nicht. So wirkt der aus Tumoren stammende VEGF nicht als autokriner mitogener Faktor. Der VEGF trägt daher in vivo dadurch zum Tumorwachstum bei, dass er durch seine parakrine Gefäßendothelzellen-Chemotaxis- und -Mitogeneseaktivität die Angiogenese fördert. Diese monoklonalen Antikörper hemmen auch das Wachstum von typischerweise weniger stark vaskularisierten Human-Kolonkarzinomen bei thymuslosen Mäusen und verringern die Anzahl der aus inokulierten Zellen entstehenden Tumore.
Die Expression eines VEGF-bindenden Konstrukts von Flk-1, Flt-1, dem zur Entfernung der zytoplasmatischen Tyrosinkinasedomänen, jedoch unter Beibehaltung eines Membranankers, verkürzten Maus-KDR-Rezeptorhomologs, in Viren stoppt praktisch das Wachstum eines transplantierbaren Glioblastoms bei der Maus, vermutlich aufgrund des dominant-negativen Mechanismus der Heterodimerbildung mit transmembranösen Endothelzellen-VEGF-Rezeptoren. Embryostammzellen, die in der Nacktmaus üblicherweise in Form von festen Tumoren wachsen, bilden bei Knock-out aller beider VEGF-Allele keine nachweisbaren Tumore. Aus diesen Daten gemeinsam geht die Rolle des VEGF beim Wachstum fester Tumore hervor. Die Hemmung von KDR bzw. Flt-1 ist an der pathologischen Angiogenese beteiligt, und diese Rezeptoren eignen sich zur Behandlung von Krankheiten, bei denen Angiogenese einen Teil der Gesamtpathologie, z.B. Entzündung, diabetische Retina-Vaskularisierung sowie verschiedene Formen von Krebs, darstellt, da bekannt ist, dass das Tumorwachstum angiogeneseabhängig ist (Weidner et al., N. Engl. J. Med., 324, S. 1-8, 1991).

Bei Angiopoietin 1 (Ang1), einem Liganden für die endothelspezifische Rezeptor-Tyrosinkinase TIE-2, handelt es sich um einen neuen angiogenen Faktor (Davis et al, Cell, 1996, 87:1161-1169; Partanen et al, Mol. Cell Biol., 12:1698-1707 (1992); US-Patent Nr. 5,521,073; 5,879,672; 5,877,020; und 6,030,831). Das Akronym TIE steht für "Tyrosinkinase mit Ig- und EGF-Homologiedomänen". TIE wird zur Identifizierung einer Klasse von Rezeptor-Tyrosinkinasen verwendet, die ausschließlich in Gefäßendothelzellen und frühen hämopoietischen Zellen exprimiert werden. TIE-Rezeptorkinasen sind typischerweise durch das Vorhandensein einer EGF-ähnlichen Domäne und einer Immunglobulin (IG)-ähnlichen Domäne charakterisiert, die aus extrazellulären Faltungseinheiten, die durch Disulfidbrückenbindungen zwischen den Ketten stabilisiert sind, besteht (Partanen et al Curr. Topics Microbiol. Immunol., 1999, 237:159-172). Im Gegensatz zu VEGF, der seine Funktion während der frühen Stadien in der Gefäßentwicklung ausübt, wirken Ang1 und sein Rezeptor TIE-2 während der späteren Stadien in der Gefäßentwicklung, d.h. während der Gefäßumbildung (Umbildung bezieht sich auf die Bildung eines Gefäßlumens) und Reifung (Yancopoulos et al, Cell, 1998, 93:661-664; Peters, K.G., Circ. Res., 1998, 83(3):342-3; Suri et al, Cell 87, 1171-1180 (1996)).

Demzufolge würde man erwarten, daß eine Hemmung von TIE-2 die Umbildung und Reifung eines durch Angiogenese initiierten neuen Gefäßsystems und dadurch den Angiogeneseprozeß unterbrechen sollte. Weiterhin würde eine Hemmung an der Kinasedomäne-Bindungsstelle von VEGFR-2 die Phosphorylierung von Tyrosinresten blockieren und dazu dienen, die Initiation der Angiogenese zu unterbrechen. Daher darf man annehmen, daß die Hemmung von TIE-2 und/oder VEGFR-2 die Tumorangiogenese verhindern und dazu dienen sollte, das Tumorwachstum zu verlangsamen oder vollständig zu beseitigen. Dementsprechend könnte man eine Behandlung von Krebs und anderen mit unangemessener Angiogenese einhergehenden Erkrankungen bereitstellen.

Die vorliegende Erfindung umfasst die Verwendung der Verbindungen der Formel I und/oder ihre physiologisch unbedenklichen Salze und Solvate zur Herstellung eines Arzneimittels zur Regulation, Modulation oder Hemmung der TIE-2 zur Vorbeugung und/oder Behandlung von Erkrankungen im Zusammenhang mit unregulierter oder gestörter TIE-2-Aktivität. Insbesondere lassen sich die Verbindungen der Formel I auch bei der Behandlung gewisser Krebsformen einsetzen. Weiterhin können die Verbindungen der Formel I verwendet werden, um bei gewissen existierenden Krebschemotherapien additive oder synergistische Effekte bereitzustellen, und/oder können dazu verwendet werden, um die Wirksamkeit gewisser existierender Krebschemotherapien und -bestrahlungen wiederherzustellen.

Weiterhin können die Verbindungen der Formel I zur Isolierung und zur Untersuchung der Aktivität oder Expression von TIE-2 verwendet werden. Außerdem eigenen sie sich insbesondere zur Verwendung in diagnostischen Verfahren zu Erkrankungen im Zusammenhang mit unregulierter oder gestörter TIE-2-Aktivität.

Die vorliegende Erfindung umfasst die Verwendung der Verbindungen der Formel I und/oder ihre physiologisch unbedenklichen Salze und Solvate zur Herstellung eines Arzneimittels zur Regulation, Modulation oder Hemmung des VEGFR-2 zur Vorbeugung und/oder Behandlung von Erkrankungen im Zusammenhang mit unregulierter oder gestörter VEGFR-2-Aktivität.

Die vorliegende Erfindung betrifft weiterhin die Verbindungen der Formel I als Inhibitoren von Raf-Kinasen.

Protein-Phosphorylierung ist ein fundamentaler Prozess für die Regulation von Zellfunktionen. Die koordinierte Wirkung von sowohl Proteinkinasen als auch Phosphatasen kontrolliert die Phosphorylierungsgrade und folglich die Aktivität spezifischer Zielproteine. Eine der vorherrschenden Rollen der Protein-Phosphorylierung ist bei der Signaltransduktion, wenn extrazelluläre Signale amplifiziert und durch eine Kaskade von Protein-Phosphorylierungs- und Dephosphorylierungsereignissen, z. B. im p21^{ras}/raf-Weg propagiert werden.

Das p21^{ras}-Gen wurde als ein Onkogen der Harvey- und Kirsten-Ratten-Sarkom-Viren (H-Ras bzw. K-Ras) entdeckt. Beim Menschen wurden charakteristische Mutationen im zellulären Ras-Gen (c-Ras) mit vielen verschiedenen Krebstypen in Verbindung gebracht. Von diesen mutanten Allelen, die Ras konstitutiv aktiv machen, wurde gezeigt, dass sie Zellen, wie zum Beispiel die murine Zelllinie NIH 3T3, in Kultur transformieren.

Das p21^{ras}-Onkogen ist ein wichtiger beitragender Faktor bei der Entwicklung und Progression humaner solider Karzinome und ist bei 30 % aller humaner Karzinome mutiert (Bolton et al. (1994) Ann. Rep. Med. Chem., 29, 165-74; Bos. (1989) Cancer Res., 49, 4682-9). In seiner normalen, nicht mutierten Form ist das Ras-Protein ein Schlüsselelement der Signaltransduktionskaskade, die durch Wachstumsfaktor-Rezeptoren in fast allen Geweben gesteuert wird (Avruch et al. (1994) Trends Biochem. Sci., 19, 279-83).

Biochemisch ist Ras ein Guanin-Nukleotid-bindendes Protein, und das Zyklieren zwischen einer GTP-gebundenen aktivierten und einer GDPgebundenen ruhenden Form wird von Ras-endogener GTPase-Aktivität und anderen Regulatorproteinen strikt kontrolliert. Das Ras-Genprodukt bindet an Guanintriphosphat (GTP) und Guanindiphosphat (GDP) und hydrolysiert GTP zu GDP. Ras ist im GTP-gebundenen Zustand aktiv. In den Ras-Mutanten in Krebszellen ist die endogene GTPase-Aktivität abgeschwächt, und folglich gibt das Protein konstitutive Wachstumssignale an "Downstream"-Effektoren, wie zum Beispiel an das Enzym Raf-Kinase ab. Dies führt zum krebsartigen Wachstum der Zellen, die diese Mutanten tragen (Magnuson et al. (1994) Semin. Cancer Biol., 5, 247-53). Das Ras-Proto-Onkogen benötigt ein funktionell intaktes C-Raf-1-Proto-Onkogen, um in höheren Eukaryoten durch Rezeptor- und Nicht-Rezeptor-Tyrosin-Kinasen initiierte Wachstums- und Differenzierungssignale zu transduzieren.

Aktiviertes Ras ist für die Aktivierung des C-Raf-1-Proto-Onkogens notwendig, die biochemischen Schritte, durch die Ras die Raf-1-Protein-(Ser/Thr)-Kinase aktiviert, sind jedoch inzwischen gut charakterisiert. Es wurde gezeigt, dass das Inhibieren des Effekts von aktivem Ras durch Inhibition des Raf-Kinase-Signalwegs mittels Verabreichung von deaktivierenden Antikörpern gegen Raf-Kinase oder mittels Koexpression dominanter negativer Raf-Kinase oder dominanter negativer MEK (MAPKK), dem Substrat der Raf-Kinase, zur Reversion transformierter Zellen zum normalen Wachstumsphänotyp führt, siehe: Daum et al. (1994) Trends Biochem. Sci., 19, 474-80; Fridman et al. (1994) J Biol. Chem., 269, 30105-8. Kolch et al. (1991) Nature, 349, 426-28) und zur Besprechung Weinstein-Oppenheimer et al. Pharm. & Therap. (2000), 88, 229-279.

Auf ähnliche Weise wurde die Inhibition von Raf-Kinase (durch Antisense-Oligodesoxynukleotide) in vitro und in vivo mit der Inhibition des Wachstums einer Reihe verschiedener humaner Tumortypen in Beziehung gebracht (Monia et al., Nat. Med. 1996, 2, 668-75).

Raf-Serin- und Threonin-spezifische Protein-Kinasen sind cytosolische Enzyme, die das Zellwachstum in einer Reihe verschiedener Zellsysteme stimulieren (Rapp, U.R., et al. (1988) in The Oncogene Handbook; T. Curran, E.P. Reddy und A. Skalka (Hrsg.) Elsevier Science Publishers; Niederlande, S. 213-253; Rapp, U.R., et al. (1988) Cold Spring Harbor Sym. Quant. Biol. 53:173-184; Rapp, U.R., et al. (1990) Inv Curr. Top. Microbiol. Immunol. Potter und Melchers (Hrsg.), Berlin, Springer-Verlag 166:129-139).

Drei Isozyme wurden charakterisiert:
C-Raf (Raf-1) (Bonner, T.I., et al. (1986) Nucleic Acids Res. 14:1009-1015). A-Raf (Beck, T.W., et al. (1987) Nucleic Acids Res. 15:595-609), und B-Raf (Qkawa, S., et al. (1998) Mol. Cell. Biol. 8:2651-2654; Sithanandam, G. et al. (1990) Oncogene:1775). Diese Enzyme unterscheiden sich durch ihre Expression in verschiedenen Geweben. Raf-1 wird in allen Organen und in allen Zelllinien, die untersucht wurden, exprimiert, und A- und B-Raf werden in Urogenital- bzw. Hirngeweben exprimiert (Storm, S.M. (1990) Oncogene 5:345-351).
Raf-Gene sind Proto-Onkogene: Sie können die maligne Transformation von Zellen initiieren, wenn sie in spezifisch veränderten Formen exprimiert werden. Genetische Veränderungen, die zu onkogener Aktivierung führen, erzeugen eine konstitutiv aktive Proteinkinase durch Entfernung oder Interferenz mit einer N-terminalen negativen Regulatordomäne des Proteins (Heidecker, G., et al. (1990) Mol. Cell. Biol. 10:2503-2512; Rapp, U.R., et al. (1987) in Oncogenes and Cancer; S. A. Aaronson, J. Bishop, T. Sugimura, M. Terada, K. Toyoshima und P. K. Vogt (Hrsg.) Japan Scientific Press, Tokyo). Mikroinjektion in NIH 3T3-Zellen von onkogen aktivierten, aber nicht Wildtyp-Versionen des mit Expressionsvektoren von Escherichia coli präparierten Raf-Proteins führt zu morphologischer Transformation und stimuliert die DNA-Synthese (Rapp, U.R., et al. (1987) in Oncogenes and Cancer; S. A. Aaronson, J. Bishop, T. Sugimura, M. Terada, K. Toyoshima, und P. K. Vogt (Hrsg.) Japan Scientific Press, Tokyo; Smith, M. R., et al. (1990) Mol. Cell. Biol. 10:3828-3833).

Folglich ist aktiviertes Raf-1 ein intrazellulärer Aktivator des Zellwachstums. Raf-1-Protein-Serin-Kinase ist ein Kandidat für den "Downstream"-Effektor der Mitogen-Signaltransduktion, da Raf-Onkogene dem Wachstumsarrest begegnen, der aus einer Blockade zellulärer Ras-Aktivität aufgrund einer zellulären Mutation (Ras-revertante Zellen) oder Mikroinjektion von Anti-Ras-Antikörpern resultiert (Rapp, U.R., et al. (1988) in The Oncogene Handbook, T. Curran, E.P. Reddy und A. Skalka (Hrsg.), Elsevier Science Publishers; Niederlande, S. 213-253; Smith, M.R., et al. (1986) Nature (London) 320:540-543).

Die C-Raf-Funktion ist für die Transformation durch eine Reihe verschiedener Membran-gebundener Onkogene und für die Wachstumsstimulation durch in Sera enthaltene Mitogene erforderlich (Smith, M.R., et al. (1986) Nature (London) 320:540-543). Raf-1-Protein-Serin-Kinase-Aktivität wird durch Mitogene über die Phosphorylierung reguliert (Morrison, D.K., et al. (1989) Cell 58:648-657), welche auch die subzelluläre Verteilung bewirkt (Olah, Z., et al. (1991) Exp. Brain Res. 84:403; Rapp, U.R., et al. (1988) Cold Spring Harbor Sym. Quant. Biol. 53:173-184. Zu Raf-1-aktivierenden Wachstumsfaktoren zählen der aus Thrombozyten stammende Wachstumsfaktor (PDGF) (Morrison, D.K., et al. (1988) Proc. Natl. Acad. Sci. USA 85:8855-8859), der Kolonien-stimulierende Faktor (Baccarini, M., et al. (1990) EMBO J. 9:3649-3657), Insulin (Blackshear, P.J., et al. (1990) J. Biol. Chem. 265:12115-12118), der epidermale Wachstumsfaktor (EGF) (Morrison, R.K., et al. (1988) Proc. Natl. Acad. Sci. USA 85:8855-8859), Interleukin-2 (Turner, B.C., et al. (1991) Proc. Natl. Acad. Sci. USA 88:1227) und Interleukin-3 und der Granulozyten-Makrophagen-Kolonien-stimulierende Faktor (Carroll, M.P., et al. (1990) J. Biol. Chem. 265:19812-19817).

Nach der Mitogen-Behandlung von Zellen transloziert die transient aktivierte Raf-1-Protein-Serin-Kinase in den perinukleären Bereich und den Nukleus (Olah, Z., et al. (1991) Exp. Brain Res. 84:403; Rapp, U.R., et al. (1988) Cold Spring Habor Sym. Quant. Biol. 53:173-184). Zellen, die aktiviertes Raf enthalten, sind in ihrem Genexpressionsmuster verändert (Heidecker, G., et al. (1989) in Genes and signal transduction in multistage carcinogenesis, N. Colburn (Hrsg.), Marcel Dekker, Inc., New York, S. 339-374) und Raf-oncogenes activate transcription from Ap-I/PEA3-dependent promotors in transient transfection assays (Jamal, S., et al. (1990) Science 344:463-466; Kaibuchi, K., et al. (1989) J. Biol. Chem. 264:20855-20858; Wasylyk, C., et al. (1989) Mol. Cell. Biol. 9:2247-2250).

Es gibt mindestens zwei unabhängige Wege für die Raf-1-Aktivierung durch extrazelluläre Mitogene: Einen, der Proteinkinase C (KC) beinhaltet, und einen zweiten, der durch Protein-Tyrosin-Kinasen initiiert wird (Blackshear, P.J., et al. (1990) J. Biol. Chem. 265:12131-12134; Kovacina, K.S., et al. (1990) J. Biol. Chem. 265:12115-12118; Morrison, D.K., et al. (1988) Proc. Natl. Acad. Sci. USA 85:8855-8859; Siegel, J.N., et al. (1990) J. Biol. Chem. 265:18472-18480; Turner, B.C., et al. (1991) Proc. Natl. Acad. Sci. USA 88:1227). In jedem Fall beinhaltet die Aktivierung Raf-1-Protein-Phosphorylierung. Raf-1-Phosphorylierung kann eine Folge einer Kinase-Kaskade sein, die durch Autophosphorylierung amplifiziert wird, oder kann vollkommen durch Autophosphorylierung hervorgerufen werden, die durch Bindung eines vermutlichen Aktivierungsliganden an die Raf-1-Regulatordomäne, analog zur PKC-Aktivierung durch Diacylglycerol initiiert wird (Nishizuka, Y. (1986) Science 233:305-312).

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279).

Die Synthese von kleinen Verbindungen, die die Signaltransduktion der Tyrosinkinasen und/oder Raf-Kinasen spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Insbesondere zeigen sie inhibierende Eigenschaften der Tyrosinkinase. Es wurde weiterhin gefunden, daß die erfindungsgemäßen Verbindungen Inhibitoren des Enzyms Raf-Kinase sind. Da das Enzym ein "Downstream"- Effektor von p21^{ras} ist, erweisen sich die Inhibitoren in pharmazeutischen Zusammensetzungen für die human- oder veterinärmedizinische Anwendung als nützlich, wenn Inhibition des Raf-Kinase-Weges, z. B. bei der Behandlung von Tumoren und/oder durch Raf-Kinase vermitteltem krebsartigen Zellwachstum, angezeigt ist. Die Verbindungen sind insbesondere nützlich bei der Behandlung solider Karzinome bei Mensch und Tier, z. B. von murinem Krebs, da die Progression dieser Krebse abhängig ist von der Ras-Protein-Signaltransduktionskaskade und deshalb auf die Behandlung durch Unterbrechung der Kaskade, d. h. durch Inhibition der Raf-Kinase, anspricht. Dementsprechend wird die erfindungsgemäßen Verbindung oder ein pharmazeutisch unbedenkliches Salz davon für die Behandlung von Krankheiten verabreicht, die durch den Raf-Kinase-Weg vermittelt werden, besonders Krebs, einschließlich solider Karzinome, wie zum Beispiel Karzinome (z. B. der Lungen, des Pankreas, der Schilddrüse, der Harnblase oder des Kolons), myeloische Erkrankungen (z. B. myeloische Leukämie) oder Adenome (z. B. villöses Kolonadenom), pathologische Angiogenese und metastatische Zellmigration. Die Verbindungen sind ferner nützlich bei der Behandlung der Komplementaktivierungsabhängigen chronischen Entzündung (Niculescu et al. (2002) Immunol. Res., 24:191-199) und durch HIV-1 (Human Immunodeficiency Virus Typ 1) induzierte Immunschwäche (Popik et al. (1998) J Virol, 72: 6406-6413).

Es wurde überraschend gefunden, daßs die erfindungsgemäßen Verbindungen mit Signalwegen, besonders mit den hierin beschriebenen Signalwegen und bevorzugt dem Raf-Kinase-Signalweg interagieren können. Die erfindungsgemäßen Verbindungen zeigen bevorzugt eine vorteilhafte biologische Aktivität, die in auf Enzymen basierenden Assays, zum Beispiel Assays wie hierin beschrieben, leicht nachweisbar ist. In derartigen auf Enzymen basierenden Assays zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Wie hierin besprochen, sind diese Signalwege für verschiedene Erkrankungen relevant. Dementsprechend sind die erfindungsgemäßen Verbindungen nützlich bei der Prophylaxe und/oder Behandlung von Erkrankungen, die von den genannten Signalwegen durch Interaktion mit einem oder mehreren der genannten Signalwege abhängig sind. Gegenstand der vorliegenden Erfindung sind deshalb erfindungsgemäße Verbindungen als Promotoren oder Inhibitoren, bevorzugt als Inhibitoren der hierin beschriebenen Signalwege. Bevorzugter Gegenstand der Erfindung sind deshalb erfindungsgemäße Verbindungen als Promotoren oder Inhibitoren, bevorzugt als Inhibitoren des Raf-Kinase-Weges. Ein bevorzugter Gegenstand der Erfindung sind deshalb erfindungsgemäße Verbindungen als Promotoren oder Inhibitoren, bevorzugt als Inhibitoren der Raf-Kinase. Ein noch bevorzugterer Gegenstand der Erfindung sind erfindungsgemäße Verbindungen als Promotoren oder Inhibitoren, bevorzugt als Inhibitoren einer oder mehrerer Raf-Kinasen, ausgewählt aus der Gruppe bestehend aus A-Raf, B-Raf und C-Raf-1. Ein besonders bevorzugter Gegenstand der Erfindung sind erfindungsgemäße Verbindungen als Promotoren oder Inhibitoren, bevorzugt als Inhibitoren von C-Raf-1.

Die vorliegende Erfindung umfasst die Verwendung der Verbindungen der Formel I und/oder ihre physiologisch unbedenklichen Salze und Solvate zur Herstellung eines Arzneimittels zur Behandlung und/oder Prophylaxe von Erkrankungen, bevorzugt den hier beschriebenen Erkrankungen, die durch Raf-Kinasen veruracht, vermittelt und/oder propagiert werden und insbesondere Erkrankungen, die durch Raf-Kinasen ausgewählt aus der Gruppe, bestehend aus A-Raf, B-Raf and C-Raf-1 verursacht, vermittelt und/oder propagiert werden. Gewöhnlich werden die hier besprochenen Erkrankungen in zwei Gruppen eingeteilt, in hyperproliferative und nicht hyperproliferative Erkrankungen. In diesem Zusammenhang werden Psoriasis, Arthritis, Entzündungen, Endometriose, Vernarbung, gutartige Prostatahyperplasie, immunologische Krankheiten, Autoimmunkrankheiten und Immunschwächekrankheiten als nicht

krebsartige Krankheiten angesehen, von denen Arthritis, Entzündung, immunologische Krankheiten, Autoimmunkrankheiten und Immunschwächekrankheiten gewöhnlich als nicht hyperproliferative Erkrankungen angesehen werden. In diesem Zusammenhang sind Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie als krebsartige Erkrankungen anzusehen, die alle gewöhnlich als hyperproliferative Erkrankungen angesehen werden. Insbesondere krebsartiges Zellwachstum und insbesondere durch Raf-Kinase vermitteltes krebsartiges Zellwachstum ist eine Erkrankung, die ein Ziel der vorliegenden Erfindung darstellt. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-Gonzälez, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).
Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J., unmittelbar vor der Veröffentlichung, Manuskript BJ20020786).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Die erfindungsgemäßen Verbindungen eignen sich auch als p38 Kinase-Inhibitoren.
Heteroarylharnstoffe, die p38 Kinase inhibieren sind in der WO 02/85859, WO 02/85857 WO99/32111 beschrieben.

### STAND DER TECHNIK

Andere Pyridopyrimidine sind in WO 98/08846 beschrieben. Andere Diarylharnstoffe sind in WO 00/42012, WO 02/062763 und WO 02/44156 beschrieben.

### ZUSAMMENFASSUNG DER ERFINDUNG

Die Erfindung betrifft Verbindungen der Formel I 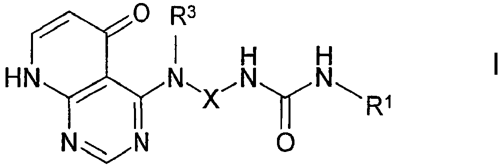 worin
- R¹: Ar oder Het¹,
- R²: A, Hal, OH, OA oder CN,
- R³, R⁴: jeweils unabhängig voneinander H oder A,
- X: unsubstituiertes oder ein-, zwei-, drei- oder vierfach durch R² substituiertes Phenylen, oder einen 6-gliedrigen aromatischen Heterocyclus mit 1-2 N-Atomen, der unsubstituiert ist oder ein-, zwei-, drei- oder vierfach durch R² substituiert sein kann,
- Ar: einen ein- oder zweikernigen aromatischen Carbocyclus mit 6-10 C-Atomen, der unsubstituiert oder ein-, zwei- oder dreifach durch Hal, A, OA, OH, Alkenyl mit 2 bis 6 C-Atomen, Alkinyl mit 2 bis 6 C-Atomen, NO₂, NH₂, NHA, NA₂, COOH, COOA, CN, Het, -O-Het, -O-Alkylen-Het, -O-Alkylen-NR³R⁴, -NR³-Alkylen-NR³R⁴, CONR³R⁴ und/oder -O-Alkylen-NR³-Alkylen-OR³ substituiert sein kann,
- Het¹: einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Hal, A, OA, OH, Alkenyl mit 2 bis 6 C-Atomen, Alkinyl mit 2 bis 6 C-Atomen, NO₂, NH₂, NHA, NA₂, COOH, COOA, CN, -O-Het, -O-Alkylen-Het, -O-Alkylen-NR³R⁴, -NR³-Alkylen-NR³R⁴, CONR³R⁴ und/oder -O-Alkylen-NR³-Alkylen-OR³ substituiert sein kann,
- Het: einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Hal, A, OA, COOA, CN und/oder Carbonylsauerstoff (=O) substituiert sein kann,
- A: Alkyl mit 1 bis 10 C-Atomen, wobei auch 1-7 H-Atome durch F und/oder Chlor ersetzt sein können,
- Hal: F, Cl, Br oder I,
bedeuten,
sowie ihre pharmazeutisch verwendbaren Derivate, Solvate, Salze, Tautomere und Stereoisomere, einschließlich deren Mischungen in allen Verhältnissen.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.
Die Formel I umfasst auch die tautomeren Verbindungen z.B. der Formel la

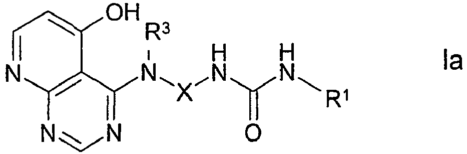

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I nach den Ansprüchen 1-10 sowie ihrer pharmazeutisch verwendbaren Derivate, Salze, Solvate, Tautomeren und Stereoisomeren, dadurch gekennzeichnet, daß man
a) eine Verbindung der Formel II 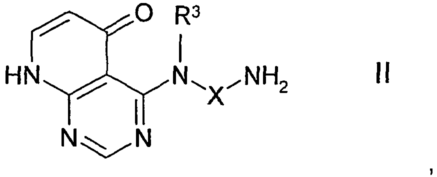 worin X und R³ die in Anspruch 1 angegebenen Bedeutungen haben,
   mit einer Verbindung der Formel III

   R¹-N=C=O III ,

   worin R¹ die in Anspruch 1 angegebene Bedeutung hat,
   umsetzt,
   oder
b) eine Verbindung der Formel II mit einer Verbindung der Formel IV

   R¹-NH₂ IV ,

   worin R¹ die in Anspruch 1 angegebene Bedeutung hat,
   und einem Chlorkohlensäureester
   umsetzt,
   und/oder
   eine Base oder Säure der Formel I in eines ihrer Salze umwandelt.

Vor- und nachstehend haben die Reste R¹, R³ und X die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl. A bedeutet auch Cycloalkyl.
Cycloalkyl bedeutet vorzugsweise Cyclopropyl, Cyclobutyl, Cylopentyl, Cyclohexyl oder Cycloheptyl.

Alkylen ist vorzugsweise unverzweigt und bedeutet bevorzugt Methylen, Ethylen, Propylen, Butylen oder Pentylen.

X bedeutet vorzugsweise Phenylen, Pyridin-diyl oder Pyrimidin-diyl, besonders bevorzugt 1,4-Phenylen, Pyridin-2,5-diyl oder Pyrimidin-2,4-diyl.

R³ und R⁴ bedeuten vorzugsweise H.

Ein ein- oder zweikerniger aromatischer Carbocyclus mit 6-10 C-Atomen bedeutet vorzugsweise Phenyl oder Naphthyl.

Ar bedeutet vorzugsweise einen ein- oder zweikernigen aromatischen Carbocyclus mit 6-10 C-Atomen, der unsubstituiert oder ein-, zwei- oder dreifach durch Hal, A, OA, OH, Het, -O-Het, -O-Alkylen-Het, -O-Alkylen-NR³R⁴, -NR³-Alkylen-NR³R⁴, CONR³R⁴ und/oder -O-Alkylen-NR³-Alkylen-OR³ substituiert sein kann.

Ar bedeutet besonders bevorzugt unsubstituiertes oder ein-, zwei- oder dreifach durch Hal, A, OA, OH, Het, -O-Alkylen-NR³R⁴, -NR³-Alkylen-NR³R⁴,-O-Het und/oder -O-Alkylen-Het substituiertes Phenyl.

Ungeachtet weiterer Substitutionen, bedeutet Het¹ z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.
Het¹ kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1 -, -2-, -3-, -4- oder -5-pyrazolyl, Tetrahydro-1-, -3- oder -4-pyrazolyl, 1,4-Dihydro-1-, -2-, -3- oder -4-pyridyl, 1 ,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinoiyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Unsubstituiertes Het¹ bedeutet besonders bevorzugt Pyridyl, Isoxazolyl, Chinolyl, Isochinolyl, Thiazolyl, [1,3,4]Thiadiazolyl, [1,2,4]Thiadiazolyl, Furyl, Thienyl, Pyrrolyl, Pyrimidinyl, Imidazolyl, Pyrazolyl, Oxazolyl, Isothiazolyl oder Pyrazinyl; ganz besonders bevorzugt Pyridyl, Isoxazolyl, Chinolyl, Isochinolyl, Thiazolyl, [1,3,4]Thiadiazolyl oder [1,2,4]Thiadiazolyl.

Ungeachtet weiterer Substitutionen, bedeutet Het z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6- , 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4.- oder -5-yl oder 2,1,3-Benzoxadiazol-5-yl.
Die heterocyclischen Reste können auch teilweise oder vollständig hydriert sein.
Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

In einer weiteren bevorzugten Ausführungsform bedeutet Het einen einkernigen gesättigten, ungesättigten oder aromatischen Heterocyclus mit 1 bis 3 N-, O- und/oder S-Atomen, der unsubstituiert ist oder einfach durch A substituiert sein kann. Der einkernige gesättigte, ungesättigte oder aromatische Heterocyclus bedeutet hierin besonders bevorzugt Piperidinyl, Pyrrolidinyl, Morpholinyl, Piperazinyl, Triazolyl, Pyridyl, Isoxazolyl, Chinolyl, Isochinolyl, Thiazolyl, [1,3,4]Thiadiazolyl, [1,2,4]Thiadiazolyl, Furyl, Thienyl, Pyrrolyl, Pyrimidinyl, Imidazolyl, Pyrazolyl, Oxazolyl, Isothiazolyl oder Pyrazinyl.

Hal bedeutet vorzugsweise F, Cl oder Br, aber auch I, besonders bevorzugt F oder Cl.

Alkenyl hat 2, 3, 4, 5 oder 6 C-Atome und steht vorzugsweise für Vinyl, 1- oder 2-Propenyl, 1-Butenyl, Isobutenyl, sek.-Butenyl, ferner bevorzugt ist 1-Pentenyl, iso-Pentenyl oder 1-Hexenyl.

Alkinyl hat 2, 3, 4, 5 oder 6 C-Atome und steht vorzugsweise für Ethinyl, Propin-1-yl, ferner für Butin-1-, Butin-2-yl, Pentin-1-, Pentin-2- oder Pentin-3-yl.
- O-Het bedeutet vorzugsweise z.B. Piperidin-4-yloxy.
- O-Alkylen-Het bedeutet vorzugsweise z.B. Morpholin-4-yl-ethoxy, Morpholin-4-yl-propoxy, 1-Methyl-piperidin-4-yl-methoxy, Piperazin-4-yl-ethoxy, Pyrrolidin-2-ylmethoxy oder Pyrrolidin-1-ylethoxy.
- O-Alkylen-NR⁸R⁹ bedeutet vorzugsweise z.B. CH₃-NH-CH₂CH₂-O-, NH₂-CH₂CH₂-O- oder (C₂H₅)₂N-CH₂CH₂-O-.
- O-Alkylen-NR⁸-Alkylen-OR⁸ bedeutet vorzugsweise z.B.
- O-CH₂CH₂-N(CH₃)-CH₂CH₂-OCH₃.

Für die gesamte Erfindung gilt, daß sämtliche Reste, die mehrfach auftreten, gleich oder verschieden sein können, d.h. unabhängig voneinander sind.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis II ausgedrückt werden, die der Formel I entsprechen und worin die nicht näher bezeichneten Reste die bei der Formel I angegebene Bedeutung haben, worin jedoch
in Ia
   - X: Phenylen, Pyridin-diyl oder Pyrimidin-diyl, bedeutet;
   in Ib
   - Ar: einen ein- oder zweikernigen aromatischen Carbocyclus mit 6-10 C-Atomen, der unsubstituiert oder ein-, zwei- oder dreifach durch Hal, A, OA, OH, Het, -O-Het, -O-Alkylen-Het, -O-Alkylen-NR³R⁴, -NR³-Alkylen-NR³R⁴, CONR³R und/oder -O-Alkylen-NR³-Alkylen-OR³ substituiert sein kann,
   bedeutet;
in Ic
   - Ar: unsubstituiertes oder ein-, zwei- oder dreifach durch Hal, A, OA, OH, -O-Alkylen-NR³R⁴, -NR³-Alkylen-NR³R⁴, Het, -O-Het und/oder -O-Alkylen-Het substituiertes Phenyl,
   bedeutet;
in Id
   - Het¹: einen einkernigen gesättigten, ungesättigten oder aromatischen Heterocyclus mit 1 bis 3 N-, O- und/oder S-Atomen, der unsubstituiert ist oder ein- oder zweifach durch Hal, A, OA und/oder OH substituiert sein kann,
   bedeutet;
in le
   - Het¹: einen einkernigen aromatischen Heterocyclus mit 1 bis 3 N-, O- und/oder S-Atomen, der unsubstituiert ist oder ein- oder zweifach durch Hal, A, OA und/oder OH substituiert sein kann,
   bedeutet;
in If
   - Het¹: einen 5- oder 6-gliedrigen aromatischen Heterocyclus mit 1 bis 2 N- und/oder O-Atomen, der unsubstituiert ist oder ein- oder zweifach durch A substituiert sein kann,
   bedeutet;
in Ig
   - Het¹: unsubstituiertes oder ein- oder zweifach durch A substituiertes Pyridyl, Isoxazolyl, Chinolyl, Isochinolyl, Thiazolyl, [1,3,4]Thiadiazolyl, [1,2,4]Thiadiazolyl, Furyl, Thienyl, Pyrrolyl, Pyrimidinyl, Imidazolyl, Pyrazolyl, Oxazolyl, Isothiazolyl oder Pyrazinyl,
   bedeutet;
in Ih
   - Het: einen einkernigen gesättigten, ungesättigten oder aromatischen Heterocyclus mit 1 bis 3 N-, O- und/oder S-Atomen, der unsubstituiert ist oder einfach durch A substituiert sein kann,
   bedeutet;
in Ii
   - Het: unsubstituiertes oder einfach durch A substituiertes Piperidinyl, Pyrrolidinyl, Morpholinyl, Piperazinyl, Triazolyl, Pyridyl, Isoxazolyl, Chinolyl, Isochinolyl, Thiazolyl, [1,3,4]Thiadiazolyl, [1,2,4]Thiadiazolyl, Furyl, Thienyl, Pyrrolyl, Pyrimidinyl, Imidazolyl, Pyrazolyl, Oxazolyl, Isothiazolyl oder Pyrazinyl,
   bedeutet;
in Ij
   - R³, R⁴: H
   bedeuten;
in Ik
   - R¹: Ar oder Het¹,
   - X: Phenylen, Pyridin-diyl oder Pyrimidin-diyl,
   - Ar: einen ein- oder zweikernigen aromatischen Carbocyclus mit 6-10 C-Atomen, der unsubstituiert oder ein-, zwei- oder dreifach durch Hal, A, OA, OH, Het, -O-Het, -O-Alkylen-Het, -O-Alkylen-NR³R⁴, -NR³-Alkylen-NR³R⁴, CONR³R⁴ und/oder -O-Alkylen-NR³-Alkylen-OR³ substituiert sein kann,
   - Het¹: einen einkernigen gesättigten, ungesättigten oder aromatischen Heterocyclus mit 1 bis 3 N-, O- und/oder S-Atomen, der unsubstituiert ist oder ein- oder zweifach durch Hal, A, OA und/oder OH substituiert sein kann,
   - Het: einen einkernigen gesättigten, ungesättigten oder aromatischen Heterocyclus mit 1 bis 3 N-, O- und/oder S-Atomen, der unsubstituiert ist oder einfach durch A substituiert sein kann,
   - A: Alkyl mit 1 bis 10 C-Atomen, wobei auch 1-7 H-Atome durch F und/oder Chlor ersetzt sein können,
   - Hal: F, Cl, Br oder I,
   bedeuten;
in Il
   - R¹: Ar oder Het¹,
   - X: Phenylen, Pyridin-diyl oder Pyrimidin-diyl,
   - Ar: unsubstituiertes oder ein-, zwei- oder dreifach durch Hal, A, OA, OH, -O-Alkylen-NR³R⁴, -NR³-Alkylen-NR³R⁴, Het, -O-Het und/oder -O-Alkylen-Het substituiertes Phenyl,
   - Het¹: Het¹ einen 5- oder 6-gliedrigen aromatischen Heterocyclus mit 1 bis 2 N- und/oder O-Atomen, der unsubstituiert ist oder ein- oder zweifach durch A substituiert sein kann,
   - Het: einen einkernigen gesättigten, ungesättigten oder aromatischen Heterocyclus mit 1 bis 3 N-, O- und/oder S-Atomen, der unsubstituiert ist oder einfach durch A substituiert sein kann,
   - A: Alkyl mit 1 bis 10 C-Atomen, wobei auch 1-7 H-Atome durch F und/oder Chlor ersetzt sein können,
   - Hal: F, Cl, Br oder I,
   bedeuten;
sowie ihre pharmazeutisch verwendbaren Derivate, Salze, Solvate, Tautomere und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Verbindungen der Formel I können vorzugsweise erhalten werden, indem man Verbindungen der Formel II mit Verbindungen der Formel III umsetzt.

Die Verbindungen der Formel II sind neu, die der Formel III sind in der Regel bekannt.

Die Umsetzung erfolgt in der Regel in einem inerten Lösungsmittel, in Gegenwart einer organischen Base wie Triethylamin, Dimethylanilin, Pyridin oder Chinolin. Die Reaktionszeit liegt je nach den angewendeten Bedingungen zwischen einigen Minuten und 14 Tagen, die Reaktionstemperatur zwischen etwa 0° und 150°, normalerweise zwischen 15° und 90°, besonders bevorzugt zwischen 15 und 30°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Verbindungen der Formel I können weiter vorzugsweise erhalten werden, indem man Verbindungen der Formel II mit Verbindungen der Formel IV und einem Chlorkohlensäureester, wie z.B. dem 4-Nitrophenylester, umsetzt. Die Verbindungen der Formel IV sind in der Regel bekannt.

Die Umsetzung erfolgt in der Regel in einem inerten Lösungsmittel, in Gegenwart eines säurebindenden Mittels vorzugsweise einer organischen Base wie DIPEA, Triethylamin, Dimethylanilin, Pyridin oder Chinolin. Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.
Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.
Als inerte Lösungsmittel eignen sich die oben genannten.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern. Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I und/oder ihre pharmazeutisch verwendbaren Derivate, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Derivate, Solvate und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Derivate, Solvate und Stereoisomere, einschließlich deren Mischungen in allen Verhältnissen,
und einer wirksamen Menge eines weiteren Arzneimittelwirkstoffs gelöst oder in lyophilisierter Form vorliegt.

### VERWENDUNG

Die vorliegenden Verbindungen eignen sich als pharmazeutische Wirkstoffe für Säugetiere, insbesondere für den Menschen, bei der Behandlung von tyrosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung der Verbindungen der Formel I und/oder ihre physiologisch unbedenklichen Salze und Solvate zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom. Ebensfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung einer Krankheit, an der Angiogenese beteiligt ist.

Eine derartige Krankheit, an der Angiogenese beteiligt ist, ist eine Augenkrankheit, wie Retina-Vaskularisierung, diabetische Retinopathie, altersbedingte Makula-Degeneration und dergleichen. Die Verwendung von Verbindungen der Formel I und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Ebenfalls umfasst ist die Verwendung der Verbindungen der Formel I und/oder ihre physiologisch unbedenklichen Salze und Solvate zur Herstellung eines Arzneimittels zur Behandlung oder Vorbeugung einer tyrosinkinasebedingten Krankheit bzw. eines tyrosinkinasebedingten Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.
Die vorliegende Erfindung umfasst auch die Verwendung Verbindungen der Formel I und/oder ihre physiologisch unbedenklichen Salze und Solvate zur Herstellung eines Arzneimittels zur Behandlung oder Vorbeugung von Retina-Vaskularisierung.
Verfahren zur Behandlung oder Vorbeugung von Augenkrankheiten wie diabetischer Retinopathie und altersbedingter Makula-Degeneration sind ebenfalls ein Bestandteil der Erfindung. Die Verwendung zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Erfindung.

Der Ausdruck "tyrosinkinasebedingte Krankheiten oder Leiden" bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubildung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Die Verbindungen der Formel I können an Patienten zur Behandlung von Krebs verabreicht werden. Die vorliegenden Verbindungen hemmen die Tumorangiogenese und beeinflussen so das Wachstum von Tumoren (J. Rak et al. Cancer Research, 55:4575-4580, 1995). Die angiogenesehemmenden Eigenschaften der vorliegenden Verbindungen der Formel I eignen sich auch zur Behandlung bestimmter Formen von Blindheit, die mit Retina-Gefäßneubildung in Zusammenhang stehen.
Die Verbindungen der Formel I eignen sich auch zur Behandlung bestimmter Knochen-Pathologien wie Osteosarkom, Osteoarthritis und Rachitis, die auch unter der Bezeichnung onkogene Osteomalazie bekannt ist (Hasegawa et al., Skeletal Radiol. 28, S.41-45, 1999; Gerber et al., Nature Medicine, Bd. 5, Nr. 6, S.623-628, Juni 1999). Da der VEGF durch den in reifen Osteoklasten exprimierten KDR/Flk-1 direkt die osteoklastische Knochenresorption fördert (FEBS Let. 473:161-164 (2000); Endocrinology, 141:1667 (2000)), eignen sich die vorliegenden Verbindungen auch zur Behandlung und Vorbeugung von Leiden, die mit Knochenresorption in Zusammenhang stehen, wie Osteoporose und Morbus Paget.
Die Verbindungen können dadurch, dass sie zerebrale Ödeme, Gewebeschädigung und ischämiebedingte Reperfusionsverletzungen reduzieren, auch zur Verringerung oder Vorbeugung von Gewebeschäden, die nach zerebralen ischämischen Ereignissen wie Gehirnschlag auftreten, verwendet werden (Drug News Perspect 11:265-270 (1998); J. Clin. Invest. 104:1613-1620 (1999)).

Gegenstand der Erfindung ist somit die Verwendung von Verbindungen der Formel I, sowie ihrer pharmazeutisch verwendbaren Derivate, Solvate und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.

Bevorzugt sind hierbei Kinasen ausgewählt aus der Gruppe der Tyrosinkinasen und Raf-Kinasen.

Vorzugsweise handelt es sich bei den Tyrosinkinasen um TIE-2, VEGFR, PDGFR, FGFR und/oder FLT/KDR.

Bevorzugt ist die Verwendung von Verbindungen der Formel I, sowie ihrer pharmazeutisch verwendbaren Derivate, Solvate und Stereoisomere, einschließlich deren Mischungen in allen Verhältnissen, zur Herstellung eines Arzneimittels zur Behandlung von Krankheiten, die durch Inhibierung der Tyrosinkinasen durch die Verbindungen nach Anspruch 1 beeinflußt werden.

Besonders bevorzugt ist die Verwendung zur Herstellung eines Arzneimittels zur Behandlung von Krankheiten, die durch Inhibierung von TIE-2, VEGFR, PDGFR, FGFR und/oder FLT/KDR durch die Verbindungen nach Anspruch 1 beeinflußt werden. Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Gegenstand der Erfindung ist weiterhin die Verwendung der Verbindungen der Formel I zur Behandlung einer Krankheit, an der Angiogenese beteiligt ist.

Vorzugsweise handelt es sich bei der Krankheit um eine Augenkrankheit.

Gegenstand der Erfindung ist weiterhin die Verwendung zur Behandlung von Retina-Vaskularisierung, diabetischer Retinopathie, altersbedingter Makula-Degeneration und/oder Entzündungskrankheiten.

Die Entzündungskrankheit ist vorzugsweise ausgewählt aus der Gruppe rheumatoide Arthritis, Schuppenflechte, Kontaktdermatitis und Spät-Typ der Überempfindlichkeitsreaktion stammt.

Gegenstand der Erfindung ist weiterhin die Verwendung der erfindungsgemäßen Verbindungen zur Behandlung von Knochen-Pathologien, wobei die Knochenpathologie aus der Gruppe Osteosarkom, Osteoarthritis und Rachitis stammt.

Die Verbindungen der Formel I eignen sich zur Herstellung eines Arzneimittels zur Behandlung von Krankheiten, die durch Raf-Kinasen verursacht, vermittelt und/oder propagiert werden, wobei die Raf-Kinase aus der Gruppe bestehend aus A-Raf, B-Raf und Raf-1 ausgewählt wird. Bevorzugt ist die Verwendung zur Behandlung von Erkrankungen, vorzugsweise aus der Gruppe der hyperproliferativen und nicht hyperproliferativen Erkrankungen.
Hierbei handelt es sich um Krebserkrankungen oder nicht krebsartige Erkrankungen.
Die nicht krebsartigen Erkrankungen sind ausgewählt aus der Gruppe bestehend aus Psoriasis, Arthritis, Entzündungen, Endometriose, Vernarbung, gutartiger Prostatahyperplasie, immunologischer Krankheiten, Autoimmunkrankheiten und Immunschwächekrankheiten.

Die krebsartigen Erkrankungen sind ausgewählt aus der Gruppe bestehend aus Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischem Krebs, Schilddrüsenkrebs, Lymphom, chronischer Leukämie und akuter Leukämie.

Die Verbindungen der Formel I können auch gemeinsam mit anderen gut bekannten Therapeutika, die aufgrund ihrer jeweiligen Eignung für das behandelte Leiden ausgewählt werden, verabreicht werden. So wären zum Beispiel bei Knochenleiden Kombinationen günstig, die antiresorptiv wirkende Bisphosphonate, wie Alendronat und Risedronat, Integrinblocker (wie sie weiter unten definiert werden), wie αvβ3-Antagonisten, bei der Hormontherapie verwendetete konjugierte Östrogene wie Prempro®, Premarin® und Endometrion®; selektive Östrogenrezeptormodulatoren (SERMs) wie Raloxifen, Droloxifen, CP-336,156 (Pfizer) und Lasofoxifen, Kathepsin-K-Hemmer und ATP-Protonenpumpenhemmer enthalten. Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. Die synergistischen Wirkungen der Hemmung des VEGF in Kombination mit Radiotherapie sind in der Fachwelt beschrieben worden (siehe WO 00/61186).
"Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1- piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Hemmer, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin,
Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis-[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyl-daunorubicin (siehe WO 00/50032), was jedoch keine Einschränkung darstellen soll.
Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNP11100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylen-dioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

Gegenstand der Erfindung ist ferner die Verwendung der Verbindungen der Formel I zur Herstellung eines Arzneimittels zur Behandlung von Krankheiten, wobei die Krankheit durch gestörte Angiogenese gekennzeichnet ist. Bei der Krankheit handelt es sich vorzugsweise um Krebserkrankungen.
Die gestörte Angiogenese resultiert vorzugsweise aus einer gestörten VEGFR-1- , VEGFR-2- und/oder VEGFR-3-Aktivität.
Besonders bevorzugt ist daher auch die Verwendung der erfindungsgemäßen Verbindungen zur Herstellung eines Arzneimittels zur Inhibierung der VEGFR-2-Aktivität.

### ASSAYS

Die in den Beispielen beschriebenen Verbindungen der Formel I wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).
VEGF-Rezeptorkinase-Assay
Die VEGF-Rezeptorkinaseaktivität wird durch Einbau von radioaktiv markiertem Phosphat in 4:1 Polyglutaminsäure/Tyrosin-Substrat (pEY) bestimmt. Das phosphorylierte pEY-Produkt wird auf einer Filtermembran festgehalten, und der Einbau des radioaktiv markierten Phosphats wird durch Szintillationszählung quantitativ bestimmt.

### MATERIALIEN

### VEGF-Rezeptorkinase

Die intrazelluläre-Tyrosinkinase-Domänen des menschlichen KDR (Terman, B. I. et al. Oncogene (1991) Bd. 6, S. 1677-1683.) und Flt-1 (Shibuya, M. et al. Oncogene (1990) Bd. 5, S. 519-524) wurden als Glutathion-S-transferase (GST)-Genfusionsproteine kloniert. Dies geschah durch Klonieren der Zytoplasma-Domäne der KDR-Kinase als leserastergerechte Fusion am Carboxy-Terminus des GST-Gens. Die löslichen rekombinanten GST-Kinasedomäne-Fusionsproteine wurden in *Spodoptera frugiperda* (Sf21) Insektenzellen (Invitrogen) unter Verwendung eines Baculovirus-Expressionsvektors (pAcG2T, Pharmingen) exprimiert.

### Lysepuffer

50 mM Tris pH 7,4, 0,5 M NaCl, 5 mM DTT, 1 mM EDTA, 0,5% Triton X-100, 10% Glycerin, je 10 mg/ml Leupeptin, Pepstatin und Aprotinin sowie 1 mM Phenylmethylsulfonylfluorid (alle von Sigma).

### Waschpuffer

50 mM Tris pH 7,4, 0,5 M NaCl, 5 mM DTT, 1 mM EDTA, 0.05% Triton X-100, 10% Glycerin, je 10 mg/ml Leupeptin, Pepstatin und Aprotinin sowie 1 mM Phenylmethylsulfonylfluorid.

### Dialysepuffer

50 mM Tris pH 7,4, 0,5 M NaCl, 5 mM DTT, 1 mM EDTA, 0.05% Triton X-100, 50% Glycerin, je 10 mg/ml Leupeptin, Pepstatin und Aprotinin sowie 1 mM Phenylmethylsulfonylfluorid.

### 10x Reaktionspuffer

200 mM Tris, pH 7,4, 1,0 M NaCl, 50 mM MnCl₂, 10 mM DTT und 5 mg/ml Rinderserumalbumin [bovine serum albumin = BSA] (Sigma).

### Enzymverdünnungspuffer

50 mM Tris, pH 7,4, 0,1 M NaCl, 1 mM DTT, 10% Glycerin, 100 mg/ml BSA.

### 10× Substrat

750 µg/ml Poly(glutaminsäure/Tyrosin; 4:1) (Sigma).

### Stopp-Lösung

30% Trichloressigsäure, 0,2 M Natriumpyrophosphat (beide von Fisher). Waschlösung
15% Trichloressigsäure, 0,2 M Natriumpyrophosphat.

### Filterplatten

Millipore #MAFC NOB, GF/C 96-Well-Glasfaserplatte.

### Verfahren A - Proteinaufreinigung

1. Die Sf21-Zellen wurden mit dem rekombinanten Virus bei einer m.o.i. (Multiplizität der Infektion) von 5 Viruspartikeln/Zelle infiziert und 48 Stunden lang bei 27°C gezüchtet.
2. Alle Schritte wurden bei 4°C durchgeführt. Die infizierten Zellen wurden durch Zentrifugieren bei 1000xg geerntet und 30 Minuten bei 4°C mit 1/10 Volumen Lysepuffer lysiert und anschließend 1 Stunde lang bei 100.000×g zentrifugiert. Der Überstand wurde dann über eine mit Lysepuffer äquilibrierte Glutathion-Sepharose-Säure (Pharmacia) gegeben und mit 5 Volumina des gleichen Puffers und anschließend 5 Volumina Waschpuffer gewaschen. Das rekombinante GST-KDR-Protein wurde mit Waschpuffer/10 mM reduziertem Glutathion (Sigma) eluiert und gegen Dialysepuffer dialysiert.

### Verfahren B - VEGF-Rezeptorkinase-Assay

1. Assay mit 5 µl Hemmstoff oder Kontrolle in 50% DMSO versetzen.
2. Mit 35 µl Reaktionsmischung, die 5 µl 10× Reaktionspuffer, 5 µl 25 mM ATP/10 µCi[³³ P]ATP (Amersham) und 5 µl 10x Substrat enthält, versetzen.
3. Reaktion durch Zugabe von 10 µl KDR (25 nM) in Enzymverdünnungspuffer starten.
4. Mischen und 15 Minuten lang bei Raumtemperatur inkubieren.
5. Reaktion durch Zugabe von 50 µl Stopp-Lösung stoppen.
6. 15 Minuten lang bei 4°C inkubieren.
7. 90-µl-Aliquot auf Filterplatte überführen.
8. Absaugen und 3 Mal mit Waschlösung waschen.
9. 30 µl Szintillations-Cocktail zugeben, Platte verschließen und in einem Szintillations-Zähler Typ Wallac Microbeta zählen.

Mitogenese-Assay an menschlichen Nabelschnurvenenendothelzellen Die Expression von VEGF-Rezeptoren, die mitogene Reaktionen auf den Wachstumsfaktor vermitteln, ist größtenteils auf Gefäßendotheizellen beschränkt. Kultivierte menschliche Nabelschnurvenenendothelzellen (HUVECs) proliferieren als Reaktion auf Behandlung mit VEGF und können als Assaysystem zur quantitativen Bestimmung der Auswirkungen von KDR-Kinasehemmern auf die Stimulation des VEGF verwendet werden. In dem beschriebenen Assay werden Einzelzellschichten von HUVECs im Ruhezustand 2 Stunden vor der Zugabe von VEGF oder "basic fibroblast growth factor" (bFGF) mit dem Konstituens oder der Testverbindung behandelt. Die mitogene Reaktion auf VEGF oder bFGF wird durch Messung des Einbaus von [³H]Thymidin in die Zell-DNA bestimmt.

### Materialien

### HUVECs

Als Primärkulturisolate tiefgefrorene HUVECs werden von Clonetics Corp bezogen. Die Zellen werden im Endothel-Wachstumsmedium (Endothelial Growth Medium = EGM; Clonetics) erhalten und in der 3. - 7. Passage für die Mitogenitätsassays verwendet.

### Kulturplatten

NUNCLON 96-Well-Polystyrol-Gewebekulturplattten (NUNC #167008). Assay-Medium

Nach Dulbecco modifiziertes Eagle-Medium mit 1 g/ml Glucose (DMEM mit niedrigem Glucosegehalt; Mediatech) plus 10% (v/v) fötales Rinderserum (Clonetics).

### Testverbindungen

Mit den Arbeitsstammlösungen der Testverbindungen wird mit 100% Dimethylsulfoxid (DMSO) solange eine Reihenverdünnung durchgeführt, bis ihre Konzentrationen um das 400-fache höher als die gewünschte Endkonzentration sind. Die letzten Verdünnungen (Konzentration 1×) werden unmittelbar vor Zugabe zu den Zellen mit Assay-Medium hergestellt.

### 10× Wachstumsfaktoren

Lösungen des menschlichen VEGF 165 (500 ng/ml; R&D Systems) und bFGF (10 ng/ml; R&D Systems) werden mit Assay-Medium hergestellt.

### 10× [³H]-Thymidin

[Methyl-³H]-Thymidin (20 Ci/mmol; Dupont-NEN) wird mit DMEM-Medium mit niedrigem Glucosegehalt auf 80 µCi/ml verdünnt.

### Zellwaschmedium

Hank's balanced salt solution (Mediatech) mit 1 mg/ml Rinderserumalbumin (Boehringer-Mannheim).

### Zell-Lyse-Lösung

1 N NaOH, 2% (w/v) Na₂CO₃.

### Verfahren 1

In EGM gehaltene HUVEC-Einzelzellschichten werden durch Trypsinbehandlung geerntet und in einer Dichte von 4000 Zellen pro 100 µl Assay-Medium pro Näpfchen in 96-Well-Platten überimpft. Das Wachstum der Zellen wird 24 Stunden bei 37°C in einer 5% CO₂ enthaltenden feuchten Atmosphäre gestoppt.

### Verfahren 2

Das Wachstumsstoppmedium wird durch 100 µl Assay-Medium ersetzt, das entweder das Konstituens (0,25% [v/v] DMSO) oder die erwünschte Endkonzentration der Testverbindung enthält. Alle Bestimmungen werden in dreifacher Wiederholung durchgeführt. Die Zellen werden dann 2 Stunden bei 37°C/5% CO₂ inkubiert, so dass die Testverbindungen in die Zellen eindringen können.

### Verfahren 3

Nach 2-stündiger Vorbehandlung werden die Zellen durch Zugabe von 10 µl Assay-Medium, 10× VEGF-Lösung oder 10x bFGF-Lösung pro Näpfchen stimuliert. Die Zellen werden dann bei 37°C/5% CO₂ inkubiert.

### Verfahren 4

Nach 24 Stunden in Anwesenheit der Wachstumsfaktoren wird mit 10x [³H]-Thymidin (10 µl/well) versetzt.

### Verfahren 5

Drei Tage nach dem Versetzen mit [³H]-Thymidin wird das Medium abgesaugt und die Zellen werden zweimal mit Zellwaschmedium gewaschen (400 µl/well, anschließend 200 µl/well). Die gewaschenen, adhärenten Zellen werden dann durch Zugabe von Zell-Lyse-Lösung (100 µl/well) und 30-minutiges Erwärmen auf 37°C solubilisiert. Die Zell-Lysate werden in 7-ml-Szintillationsrährchen aus Glas, die 150 µl Wasser enthalten, überführt. Man versetzt mit dem Szintillations-Cocktail (5 ml/Röhrchen), und die mit den Zellen assoziierte Radioaktivität wird flüssigkeitsszintillationsspektroskopisch bestimmt.

Gemäß diesen Assays stellen die Verbindungen der Formel I VEGF-Hemmer dar und eignen sich daher zur Hemmung der Angiogenese, wie bei der Behandlung von Augenkrankheiten, z.B. diabetischer Retinopathie, und zur Behandlung von Karzinomen, z.B. festen Tumoren. Die vorliegenden Verbindungen hemmen die VEGF-stimulierte Mitogenese von kultivierten menschlichen Gefäßendothelzellen mit HK50-Werten von 0,01-5,0 µM. Diese Verbindungen sind im Vergleich zu verwandten Tyrosinkinasen (z.B. FGFR1 sowie Src-Familie; zur Beziehung zwischen Src-Kinasen und VEGFR-Kinasen siehe Eliceiri et al., Molecular Cell, Bd. 4, S.915-924, Dezember 1999) auch selektiv.

Die ***TIE-2***-Tests können z.B. analog der in WO 02/44156 angegebenen Methoden durchgeführt werden.

Der Assay bestimmt die inhibierende Aktivität der zu testenden Substanzen bei der Phosphorylierung des Substrats Poly(Glu, Tyr) durch Tie-2-Kinase in Gegenwart von radioaktivem ³³P-ATP. Das phosphorylierte Substrat bindet während der Inkubationszeit an die Oberfläche einer "flashplate"-Mikrotiterplatte. Nach Entfernen der Reaktionsmischung wird mehrmals gewaschen und anschließend die Radioaktivität an der Oberfläche der Mikrotiterplatte gemessen. Ein inhibierender Effekt der zu messenden Substanzen hat eine geringere Radioaktivität, verglichen mit einer ungestörten enzymatischen Reaktion, zur Folge.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
Massenspektrometrie (MS):
   EI (Elektronenstoß-Ionisation) M⁺
   FAB (Fast Atom Bombardment) (M+H)⁺
   ESI (Electrospray lonization) (M+H)⁺
APCI-MS (atmospheric pressure chemical ionization - mass spectrometry) (M+H)⁺.

### Beispiel 1

Die Herstellung von 1-(2-Fluor-5-trifluormethyl-phenyl)-3-[4-(5-oxo-5,8-dihydro-pyrido[2,3-*d*]pyrimidin-4-ylamino)-phenyl]-harnstoff ("1") erfolgt analog nachstehendem Schema 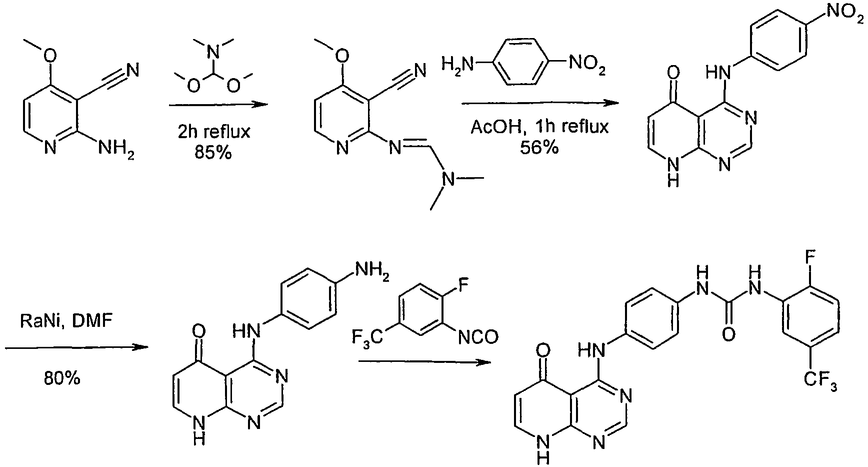
1.1 Die Herstellung von 2-Amino-4-methoxy-nicotinonitril erfolgt nach Literaturvorschrift: Jean-Luc Girardet et al. J. Med. Chem. 2000, 43, 3704-3713.
1.2 Herstellung von *N*'-(3-Cyan-4-methoxy-pyridin-2-yl)-*N*,*N*-dimethyl-formamidin:
20 g 2-Amino-4-methoxy-nicotinonitril werden mit 50 ml N,N-Dimethyl-formamiddimethylacetal zusammengegeben. Die Mischung wird unter Stickstoff 1.5 h am Rückfluss gekocht. Man läßt langsam auf Raumtemperatur abkühlen, wobei das Produkt auskristallisiert. Die Mischung wird abgesaugt und mit Ether gewaschen.
Man erhält 26 g des gewünschten Produktes, Rf 0.7 (CH₂Cl₂/MeOH 9:1).

1.3 Herstellung von 4-(4-Nitro-phenylamino)-8*H*-pyrido[2,3-d]pyrimidin-5-on:
25 g des unter 1.2 hergestellten Formamidins werden mit 20.5 g 4-Nitroanilin und 270 ml Eisessig 1 h am Rückfluss gekocht.
Das Reaktionsgemisch wird abgekühlt, mit Ether versetzt und abgesaugt.
Man erhält 20.5 g des gewünschten Produktes, Rf 0.6 (CH₂Cl₂/MeOH 9:1), HPLC-APCI-MS [M + H⁺] 284.

1.4 Herstellung von 4-(4-Amino-phenylamino)-8*H*-pyrido[2,3-d]-pyrimidin-5-on:
20 g der unter 1.3 hergestellten Nitroverbindung werden in 2 l DMF gelöst. Die Hydrierung erfolgt mit 20 g RaNi und H₂.
Die Lösung wird einrotiert, mit Methanol verrieben und abgesaugt.
Man erhält 14 g der gewünschten Verbindung,
Rf 0.25 (CH₂Cl₂/MeOH 9:1), EI-MS [M + H⁺] 254.

1.5 Herstellung von 1-(2-Fluor-5-trifluormethyl-phenyl)-3-[4-(5-oxo-5,8-dihydro-pyrido[2,3-*d*]pyrimidin-4-ylamino)-phenyl]-harnstoff ("1"):
100 mg des unter 1.4 hergestellten Amins und 0.08 ml 2-Fluor-5-(trifluormethyl)phenylisocyanant werden in 2 ml DMF gelöst. Die Mischung wird über Nacht bei Raumtemperatur gerührt. Der ausgefallene Niederschlag wird abgesaugt. Man erhält 109 mg "1", Rf 0.33 (CH₂Cl₂/MeOH 9:1), HPLC-APCI-MS [M+H⁺] 459.
Die Bedingungen für die präparative HPLC sind:

| | |
|---|---|
| Säule: | RP 18 (7 µm) Lichrosorb 250x25 |
| Fließmittel: | A: 98H2O, 2CH3CN, 0,1%TFA |
| | B: 10H2O, 90CH3CN, 0,1 %TFA |
| UV: | 225NM |
| Fluß: | 10ml/min |

### Beispiel 2

Analog Beispiel 1 erhält man die nachstehenden Verbindungen

| Nr. | Name | MW | APCI-MS |
|---|---|---|---|
| "2" | 1-(3-Trifluormethyl-phenyl)-3-[4-(5-oxo-5,8-dihydro-pyrido[2,3-*d*]pyrimidin-4-ylamino)-phenyl]-harnstoff | 440.4 | 441 |
| "3" | 1-(4-Chlor-5-trifluormethyl-phenyl)-3-[4-(5-oxo-5,8-dihydro-pyrido[2,3-*d*]pyrimidin-4-ylamino)-phenyl]-harnstoff | 474.8 | 475 |
| "4" | 1-(4-Methoxy-5-trifluormethyl-phenyl)-3-[4-(5-oxo-5,8-dihydro-pyrido[2,3-*d*]pyrimidin-4-ylamino)-phenyl]-harnstoff | 470.4 | 471 |
| "5" | 1-(3-Isopropyl-phenyl)-3-[4-(5-oxo-5,8-dihydro-pyrido[2,3-*d*]pyrimidin-4-ylamino)-phenyl]-harnstoff | 414.5 | 415 |
| "6" | 1-(2-Methoxy-5-trifluormethyl-phenyl)-3-[4-(5-oxo-5,8-dihydro-pyrido[2,3-*d*]pyrimidin-4-ylamino)-phenyl]-harnstoff | 470.4 | 471 |
| "7" | 1-(3-Fluor-5-trifluormethyl-phenyl)-3-[4-(5-oxo-5,8-dihydro-pyrido[2,3-*d*]pyrimidin-4-ylamino)-phenyl]-harnstoff | 458.4 | 459 |
| "8" | 1-(4-Fluor-5-trifluormethyl-phenyl)-3-[4-(5-oxo-5,8-dihydro-pyrido[2,3-*d*]pyrimidin-4-ylamino)-phenyl]-harnstoff | 458.4 | 459 |
| "9" | 1-(3-Trifluormethoxy-phenyl)-3-[4-(5-oxo-5,8-dihydro-pyrido[2,3-*d*]pyrimidin-4-ylamino)-phenyl]-harnstoff | 456.4 | 457 |
| "10" | 1-[2-(3-Morpholin-4-yl-propoxy)-5-trifluormethyl-phenyl]-3-[4-(5-oxo-5,8-dihydro-pyrido[2,3-d]-pyrimidin-4-ylamino)-phenyl]-harnstoff | 583.6 | 584 |
| "11" | 1-[4-(3-Morpholin-4-yl-propoxy)-5-trifluormethyl-phenyl]-3-[4-(5-oxo-5,8-dihydro-pyrido[2,3-*d*]-pyrimidin-4-ylamino)-phenyl]-harnstoff | 583.6 | 584 |
| "12" | 1-(3-Isopropoxy-phenyl)-3-[4-(5-oxo-5,8-dihydro-pyrido[2,3-*d*]pyrimidin-4-ylamino)-phenyl]-harnstoff | 430.5 | 431 |
| "13" | 1-(3-Trifluormethyl-phenyl)-3-[2-(5-oxo-5,8-dihydro-pyrido[2,3-*d*]pyrimidin-4-ylamino)-pyridin-5-yl]-harnstoff | | |
| "14" | 1-(3-Trifluormethyl-phenyl)-3-[4-(5-oxo-5,8-dihydro-pyrido[2,3-*d*]copyrimidin-4-ylamino)-phenyl]-harnstoff | | |
| "15" | 1-[2-(1,2,4-Triazol-1-yl)-5-trifluormethyl-phenyl]-3-[4-(5-oxo-5,8-dihydro-pyrido[2,3-*d*]pyrimidin-4-ylamino)-phenyl]-harnstoff | | |
| "16" | 1-(5-tert.-Butyl-isoxazol-3-yl)-3-[4-(5-oxo-5,8-dihydro-pyrido[2,3-*d*]pyrimidin-4-ylamino)-phenyl]-harnstoff | | |
| "17" | 1-(3-Trifluormethyl-phenyl)-3-[2-(5-oxo-5,8-dihydro-pyrido[2,3-*d*]pyrimidin-4-ylamino)-pyrimidin-5-yl]-harnstoff | | |
| "18" | 1-(3-Ethyl-phenyl)-3-[4-(5-oxo-5,8-dihydro-pyrido[2,3-*d*]pyrimidin-4-ylamino)-phenyl]-harnstoff | | |
| "19" | 1-[6-(1-Methyl-piperidin-4-yloxy)-3-trifluormethyl-phenyl]-3-[2-(5-oxo-5,8-dihydro-pyrido[2,3-*d*]pyrimidin-4-ylamino)-pyridin-5-yl]-harnstoff | | |
| "20" | 1-[6-(1-Methyl-piperidin-4-yl-methoxy)-3-trifluormethyl-phenyl]-3-[2-(5-oxo-5,8-dihydro-pyrido[2,3-*d*]pyrimidin-4-ylamino)-pyridin-5-yl]-harnstoff | | |
| "21" | 1-(3-Trifluormethyl-phenyl)-3-[3-(5-oxo-5,8-dihydro-pyrido[2,3-*d*]pyrimidin-4-ylamino)-phenyl]-harnstoff | | |
| "22" | 1-(3-Isopropyl-phenyl)-3-[3-(5-oxo-5,8-dihydro-pyrido[2,3-*d*]pyrimidin-4-ylamino)-phenyl]-harnstoff | | |
| "23" | 1-(3-Trifluormethoxy-phenyl)-3-[3-(5-oxo-5,8-dihydro-pyrido[2,3-*d*]pyrimidin-4-ylamino)-phenyl]-harnstoff | | |
| "24" | 1-[4-(1-Methyl-piperidin-4-y(oxy)-3-trifluormethyl-phenyl]-3-[4-(5-oxo-5,8-dihydro-pyrido[2,3-d]pyrimidin-4-ylamino)-phenyl]-harnstoff | 553.5 | 554 |
| "25" | 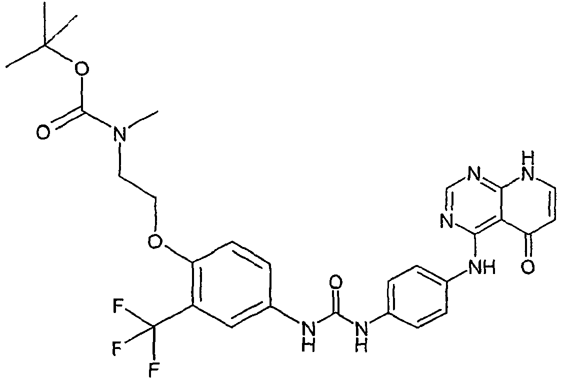 | 613.6 | 614 |
| "26" | aus "25" durch Abspaltung der BOC-Gruppe (Dichlormethan/TFA): 1-[4-(2-Methylamino-ethoxy)-3-trifluormethyl-phenyl]-3-[4-(5-oxo-5,8-dihydro-pyrido[2,3-*d*]pyrimidin-4-ylamino)-phenyl]-harnstoff | 513.5 | 514 |

Die nachfolgenden Beispiele betreffen Arzneimittel:

### Beispiel A: Injektionsgläser

Eine Lösung von 100 g eines Wirkstoffes der Formel 1 und 5 g Dinatriumhydrogenphosphat wird in 3 l zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 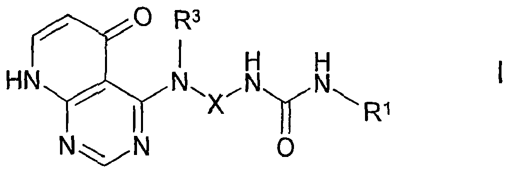 worin
R¹ Ar oder Het¹,
R² A, Hal, OH, OA oder CN,
R³, R⁴ jeweils unabhängig voneinander H oder A,
X unsubstituiertes oder ein-, zwei-, drei- oder vierfach durch R² substituiertes Phenylen, oder einen 6-gliedrigen aromatischen Heterocyclus mit 1-2 N-Atomen, der unsubstituiert ist oder ein-, zwei-, drei- oder vierfach durch R² substituiert sein kann,
Ar einen ein- oder zweikernigen aromatischen Carbocyclus mit 6-10 C-Atomen, der unsubstituiert oder ein-, zwei- oder dreifach durch Hal, A, OA, OH, Alkenyl mit 2 bis 6 C-Atomen, Alkinyl mit 2 bis 6 C-Atomen, NO₂, NH₂, NHA, NA₂, COOH, COOA, CN, Het, -O-Het, -O-Alkylen-Het, -O-Alkylen-NR³R⁴, -NR3-Alkylen-NR³R⁴, CONR³R⁴ und/oder -O-Alkylen-NR³-Alkylen-OR³ substituiert sein kann,
Het¹ einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Hal, A, OA, OH, Alkenyl mit 2 bis 6 C-Atomen, Alkinyl mit 2 bis 6 C-Atomen, NO₂, NH₂, NHA, NA₂, COOH, COOA, CN, -O-Het, -O-Alkylen-Het, -O-Alkylen-NR³R⁴, -NR³-Alkylen-NR³R⁴, CONR³R⁴ und/oder -O-Alkylen-NR³-Alkylen-OR³ substituiert sein kann,
Het einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Hal, A, OA, COOA, CN und/oder Carbonylsauerstoff (=O) substituiert sein kann, A Alkyl mit 1 bis 10 C-Atomen, wobei auch 1-7 H-Atome durch F und/oder Chlor ersetzt sein können,
Hal F, Cl, Br oder I,
bedeuten,
sowie ihre pharmazeutisch verwendbaren Derivate, Solvate, Salze, Tautomere und Stereoisomere, einschließlich deren Mischungen in allen Verhältnissen.

2. Verbindungen nach Anspruch 1, worin
X Phenylen, Pyridin-diyl oder Pyrimidin-diyl,
bedeutet,
sowie ihre pharmazeutisch verwendbaren Derivate, Solvate, Salze, Tautomere und Stereoisomere, einschließlich deren Mischungen in allen Verhältnissen.

3. Verbindungen nach Anspruch 1 oder 2, worin
Ar einen ein- oder zweikernigen aromatischen Carbocyclus mit 6-10 C-Atomen, der unsubstituiert oder ein-, zwei- oder dreifach durch Hal, A, OA, OH, Het, -O-Het, -O-Alkylen-Het, -O-Alkylen-NR³R⁴, -NR³-Alkylen-NR³R⁴, CONR³R⁴ und/oder -O-Alkylen-NR³-Alkylen-OR³ substituiert sein kann,
bedeutet, sowie ihre pharmazeutisch verwendbaren Derivate, Solvate, Salze, Tautomere und Stereoisomere, einschließlich deren Mischungen in allen Verhältnissen.

4. Verbindungen nach einem oder mehreren der Ansprüche 1-3, worin
Ar unsubstituiertes oder ein-, zwei- oder dreifach durch Hal, A, OA, OH, -O-Alkylen-NR³R⁴, -NR³-Alkylen-NR³R⁴, Het, -O-Het und/oder -O-Alkylen-Het substituiertes Phenyl,
bedeutet,
sowie ihre pharmazeutisch verwendbaren Derivate, Solvate, Salze, Tautomere und Stereoisomere, einschließlich deren Mischungen in allen Verhältnissen.

5. Verbindungen nach einem oder mehreren der Ansprüche 1-4, worin
Het¹ einen einkernigen gesättigten, ungesättigten oder aromatischen Heterocyclus mit 1 bis 3 N-, O- und/oder S-Atomen, der unsubstituiert ist oder ein- oder zweifach durch Hal, A, OA und/oder OH substituiert sein kann,
bedeutet,
sowie ihre pharmazeutisch verwendbaren Derivate, Solvate, Salze, Tautomere und Stereoisomere, einschließlich deren Mischungen in allen Verhältnissen.

6. Verbindungen nach einem oder mehreren der Ansprüche 1-5, worin
Het¹ einen einkernigen aromatischen Heterocyclus mit 1 bis 3 N-, O- und/oder S-Atomen, der unsubstituiert ist oder ein- oder zweifach durch Hal, A, OA und/oder OH substituiert sein kann,
bedeutet, sowie ihre pharmazeutisch verwendbaren Derivate, Solvate, Salze, Tautomere und Stereoisomere, einschließlich deren Mischungen in allen Verhältnissen.

7. Verbindungen nach einem oder mehreren der Ansprüche 1-6, worin
Het¹ einen 5- oder 6-gliedrigen aromatischen Heterocyclus mit 1 bis 2 N- und/oder O-Atomen, der unsubstituiert ist oder ein- oder zweifach durch A substituiert sein kann,
bedeutet,
sowie ihre pharmazeutisch verwendbaren Derivate, Solvate, Salze, Tautomere und Stereoisomere, einschließlich deren Mischungen in allen Verhältnissen.

8. Verbindungen nach einem oder mehreren der Ansprüche 1-7, worin
Het¹ unsubstituiertes oder ein- oder zweifach durch A substituiertes Pyridyl, Isoxazolyl, Chinolyl, Isochinolyl, Thiazolyl, [1,3,4]Thiadiazolyl, [1,2,4]Thiadiazolyl, Furyl, Thienyl, Pyrrolyl, Pyrimidinyl, Imidazolyl, Pyrazolyl, Oxazolyl, Isothiazolyl oder Pyrazinyl,
bedeutet,
sowie ihre pharmazeutisch verwendbaren Derivate, Solvate, Salze, Tautomere und Stereoisomere, einschließlich deren Mischungen in allen Verhältnissen.

9. Verbindungen nach einem oder mehreren der Ansprüche 1-8, worin
Het einen einkernigen gesättigten, ungesättigten oder aromatischen Heterocyclus mit 1 bis 3 N-, O- und/oder S-Atomen, der unsubstituiert ist oder einfach durch A substituiert sein kann,
bedeutet,
sowie ihre pharmazeutisch verwendbaren Derivate, Solvate, Salze, Tautomere und Stereoisomere, einschließlich deren Mischungen in allen Verhältnissen.

10. Verbindungen nach einem oder mehreren der Ansprüche 1-9, worin
Het unsubstituiertes oder einfach durch A substituiertes Piperidinyl, Pyrrolidinyl, Morpholinyl, Piperazinyl, Triazolyl, Pyridyl, Isoxazolyl, Chinolyl, Isochinolyl, Thiazolyl, [1,3,4]Thiadiazolyl, [1,2,4]Thiadiazolyl, Furyl, Thienyl, Pyrrolyl, Pyrimidinyl, Imidazolyl, Pyrazolyl, Oxazolyl, Isothiazolyl oder Pyrazinyl,
bedeutet,
sowie ihre pharmazeutisch verwendbaren Derivate, Solvate, Salze, Tautomere und Stereoisomere, einschließlich deren Mischungen in allen Verhältnissen.

11. Verbindungen nach einem oder mehreren der Ansprüche 1-10, worin
R³, R⁴ H
bedeuten,
sowie ihre pharmazeutisch verwendbaren Derivate, Solvate, Salze, Tautomere und Stereoisomere, einschließlich deren Mischungen in allen Verhältnissen.

12. Verbindungen nach einem oder mehreren der Ansprüche 1-11, worin worin
R¹ Ar oder Het¹,
X Phenylen, Pyridin-diyl oder Pyrimidin-diyl,
Ar einen ein- oder zweikernigen aromatischen Carbocyclus mit 6-10 C-Atomen, der unsubstituiert oder ein-, zwei- oder dreifach durch Hal, A, OA, OH, Het, -O-Het, -O-Alkylen-Het, -O-Alkylen-NR³R⁴, -NR³-Alkylen-NR³R⁴, CONR³R⁴ und/oder -O-Alkylen-NR³-Alkylen-OR³ substituiert sein kann,
Het¹ einen einkernigen gesättigten, ungesättigten oder aromatischen Heterocyclus mit 1 bis 3 N-, O- und/oder S-Atomen, der unsubstituiert ist oder ein- oder zweifach durch Hal, A, OA und/oder OH substituiert sein kann,
Het einen einkernigen gesättigten, ungesättigten oder aromatischen Heterocyclus mit 1 bis 3 N-, O- und/oder S-Atomen, der unsubstituiert ist oder einfach durch A substituiert sein kann,
A Alkyl mit 1 bis 10 C-Atomen, wobei auch 1-7 H-Atome durch F und/oder Chlor ersetzt sein können,
Hal F, Cl, Br oder I,
bedeuten,
sowie ihre pharmazeutisch verwendbaren Derivate, Solvate, Salze, Tautomere und Stereoisomere, einschließlich deren Mischungen in allen Verhältnissen.

13. Verbindungen nach einem oder mehreren der Ansprüche 1-12, worin worin
R¹ Ar oder Het¹,
X Phenylen, Pyridin-diyl oder Pyrimidin-diyl,
Ar unsubstituiertes oder ein-, zwei- oder dreifach durch Hal, A, OA, OH, -O-Alkylen-NR³R⁴, -NR³-Alkylen-NR³R⁴, Het, -O-Het und/oder -O-Alkylen-Het substituiertes Phenyl,
Het¹ Het¹ einen 5- oder 6-gliedrigen aromatischen Heterocyclus mit 1 bis 2 N- und/oder O-Atomen, der unsubstituiert ist oder ein- oder zweifach durch A substituiert sein kann,
Het einen einkernigen gesättigten, ungesättigten oder aromatischen Heterocyclus mit 1 bis 3 N-, O- und/oder S-Atomen, der unsubstituiert ist oder einfach durch A substituiert sein kann,
A Alkyl mit 1 bis 10 C-Atomen, wobei auch 1-7 H-Atome durch F und/oder Chlor ersetzt sein können,
Hal F, Cl, Br oder I,
bedeuten,
sowie ihre pharmazeutisch verwendbaren Derivate, Solvate, Salze, Tautomere und Stereoisomere, einschließlich deren Mischungen in allen Verhältnissen.

14. Verbindungen nach Anspruch 1, ausgewählt aus der Gruppe
| | |
|---|---|
| "1" | 1-(2-Fluor-5-trifluormethyl-phenyl)-3-[4-(5-oxo-5,8-dihydro-pyrido[2,3-*d*]pyrimidin-4-ylamino)-phenyl]-harnstoff |
| "2" | 1-(3-Trifluormethyl-phenyl)-3-[4-(5-oxo-5,8-dihydro-pyrido[2,3-*d*]pyrimidin-4-ylamino)-phenyl]-harnstoff |
| "3" | 1-(4-Chlor-5-trifluormethyl-phenyl)-3-[4-(5-oxo-5,8-dihydro-pyrido[2,3-*d*]pyrimidin-4-ylamino)-phenyl]-harnstoff |
| "4" | 1-(4-Methoxy-5-trifluormethyl-phenyl)-3-[4-(5-oxo-5,8-dihydro-pyrido[2,3-*d*]pyrimidin-4-ylamino)-phenyl]-harnstoff |
| "5" | 1-(3-Isopropyl-phenyl)-3-[4-(5-oxo-5,8-dihydro-pyrido[2,3-*d*]pyrimidin-4-ylamino)-phenyl]-harnstoff |
| "6" | 1-(2-Methoxy-5-trifluormethyl-phenyl)-3-[4-(5-oxo-5,8-dihydro-pyrido[2,3-*d*]pyrimidin-4-ylamino)-phenyl]-harnstoff |
| "7" | 1-(3-Fluor-5-trifluormethyl-phenyl)-3-[4-(5-oxo-5,8-dihydro-pyrido[2,3-*d*]pyrimidin-4-ylamino)-phenyl]-harnstoff |
| "8" | 1-(4-Fluor-5-trifluormethyl-phenyl)-3-[4-(5-oxo-5,8-dihydro-pyrido[2,3-*d*]pyrimidin-4-ylamino)-phenyl]-harnstoff |
| "9" | 1-(3-Trifluormethoxy-phenyl)-3-[4-(5-oxo-5,8-dihydro-pyrido[2,3-*d*]pyrimidin-4-ylamino)-phenyl]-harnstoff |
| "10" | 1-[2-(3-Morpholin-4-yl-propoxy)-5-trifluormethyl-phenyl]-3-[4-(5-oxo-5,8-dihydro-pyrido[2,3-*d*]pyrimidin-4-ylamino)-phenyl]-harnstoff |
| "11" | 1-[4-(3-Morpholin-4-yl-propoxy)-5-trifluormethyl-phenyl]-3-[4-(5-oxo-5,8-dihydro-pyrido[2,3-*d*]pyrimidin-4-ylamino)-phenyl]-harnstoff |
| "12" | 1-(3-Isopropoxy-phenyl)-3-[4-(5-oxo-5,8-dihydro-pyrido[2,3-*d*]pyrimidin-4-ylamino)-phenyl]-harnstoff |
| "13" | 1-(3-Trifluormethyl-phenyl)-3-[2-(5-oxo-5,8-dihydro-pyrido[2,3-*d*]pyrimidin-4-ylamino)-pyridin-5-yl]-harnstoff |
| "14" | 1-(3-Trifluormethyl-phenyl)-3-[4-(5-oxo-5,8-dihydro-pyrido[2,3-*d*]pyrimidin-4-ylamino)-phenyl]-harnstoff |
| "15" | 1-[2-(1,2,4-Triazol-1-yl)-5-trifluormethyl-phenyl]-3-[4-(5-oxo-5,8-dihydro-pyrido[2,3-*d*]pyrimidin-4-ylamino)-phenyl]-harnstoff |
| "16" | 1-(5-tert.-Butyl-isoxazol-3-yl)-3-[4-(5-oxo-5,8-dihydro-pyrido[2,3-*d*]pyrimidin-4-ylamino)-phenyl]-harnstoff |
| "17" | 1-(3-Trifluormethyl-phenyl)-3-[2-(5-oxo-5,8-dihydro-pyrido[2,3-*d*]pyrimidin-4-ylamino)-pyrimidin-5-yl]-harnstoff |
| "18" | 1-(3-Ethyl-phenyl)-3-[4-(5-oxo-5,8-dihydro-pyrido[2,3-*d*]pyrimidin-4-ylamino)-phenyl]-harnstoff |
| "19" | 1-[6-(1-Methyl-piperidin-4-yloxy)-3-trifluormethyl-phenyl]-3-[2-(5-oxo-5,8-dihydro-pyrido[2,3-*d*]pyrimidin-4-ylamino)-pyridin-5-y)]-harnstoff |
| "20" | 1-[6-(1-Methyl-piperidin-4-yl-methoxy)-3-trifluormethyl-phenyl]-3-[2-(5-oxo-5,8-dihydro-pyrido[2,3-*d*]pyrimidin-4-ylamino)-pyridin-5-yl]-harnstoff |
| "21" | 1-(3-Trifluormethyl-phenyl)-3-[3-(5-oxo-5,8-dihydro-pyrido[2,3-*d*]pyrimidin-4-ylamino)-phenyl]-harnstoff |
| "22" | 1-(3-Isopropyl-phenyl)-3-[3-(5-oxo-5,8-dihydro-pyrido[2,3-*d*]pyrimidin-4-ylamino)-phenyl]-harnstoff |
| "23" | 1-(3-Trifluormethoxy-phenyl)-3-[3-(5-oxo-5,8-dihydro-pyrido[2,3-*d*]pyrimidin-4-ylamino)-phenyl]-harnstoff |
| "24" | 1-[4-(1-Methyl-piperidin-4-yloxy)-3-trifluormethyl-phenyl]-3-[4-(5-oxo-5,8-dihydro-pyrido[2,3-d]pyrimidin-4-ylamino)-phenyl]-harnstoff |
| "26" | 1-[4-(2-Methylamino-ethoxy)-3-trifluormethyl-phenyl]-3-[4-(5-oxo-5,8-dihydro-pyrido[2,3-d]pyrimidin-4-ylamino)-phenyl]-harnstoff |
sowie ihre pharmazeutisch verwendbaren Derivate, Solvate, Salze, Tautomere und Stereoisomere, einschließlich deren Mischungen in allen Verhältnissen.

15. Verfahren zur Herstellung von Verbindungen der Formel I nach den Ansprüchen 1-14 sowie ihrer pharmazeutisch verwendbaren Derivate, Salze, Solvate, Tautomeren und Stereoisomeren, **dadurch gekennzeichnet, daß** man
a) eine Verbindung der Formel II 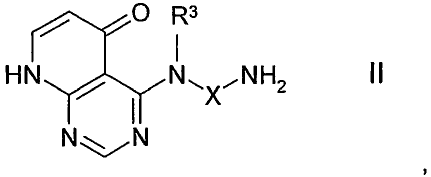 worin X und R³ die in Anspruch 1 angegebenen Bedeutungen haben,
mit einer Verbindung der Formel III
R¹-N=C=O III ,
worin R¹ die in Anspruch 1 angegebene Bedeutung hat,
umsetzt,
oder
b) eine Verbindung der Formel II mit einer Verbindung der Formel IV
R¹-NH₂ IV ,
worin R¹ die in Anspruch 1 angegebene Bedeutung hat,
und einem Chlorkohlensäureester
umsetzt,
und/oder
eine Base oder Säure der Formel I in eines ihrer Salze umwandelt.

16. Arzneimittel, enthaltend mindestens eine Verbindung der Formel I nach Anspruch 1 und/oder ihre pharmazeutisch verwendbaren Derivate, Salze, Solvate, Tautomeren und Stereoisomeren, einschließlich deren Mischungen in allen Verhältnissen, sowie gegebenenfalls Träger- und/oder Hilfsstoffe.

17. Verwendung von Verbindungen nach Anspruch 1 sowie ihrer pharmazeutisch verwendbaren Derivate, Salze, Solvate, Tautomeren und Stereoisomeren, einschließlich deren Mischungen in allen Verhältnissen,
zur Herstellung eines Arzneimittels zur Behandlung von Krankheiten, bei denen die Hemmung, Regulierung und/oder Modulation der Signaltransduktion von Kinasen eine Rolle spielt, wobei die Krankheiten ausgewähit sind aus der Gruppe fester Tumor, Tumor des Blut- und Immunsystems, Augenkrankheit, Retina-Vaskularisierung, diabetische Retinopathie, altersbedingte Makula-Degeneration, Entzündungskrankheiten, Knochen-Pathologien, wobei die Knochenpathologie aus der Gruppe Osteosarkom, Osteoarthritis und Rachitis stammt, Psoriasis, Arthritis, Entzündungen, Endometriose, Vernarbung, gutartiger Prostatahyperplasie, immunologischer Krankheiten, Autoimmunkrankheiten, Immunschwächekrankheiten.

18. Verwendung nach Anspruch 17, wobei der feste Tumor aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge stammt.

19. Verwendung nach Anspruch 17, wobei der feste Tumor aus der Gruppe Monozytenleukämie, Lungenadenokarzinom, kleinzellige Lungenkarzinome, Bauchspeicheldrüsenkrebs, Glioblastome und Brustkarzinom stammt.

20. Verwendung nach Anspruch 17, wobei der feste Tumor aus der Gruppe der Lungenadenokarzinom, kleinzellige Lungenkarzinome, Bauchspeicheldrüsenkrebs, Glioblastome, Kolonkarzinom und Brustkarzinom stammt.

21. Verwendung nach Anspruch 17, wobei der Tumor des Blut- und Immunsystems aus der Gruppe der akuten myelotischen Leukämie, der chronischen myelotischen Leukämie, akuten lymphatischen Leukämie und/oder chronischen lymphatischen Leukämie stammt.

22. Verwendung nach Anspruch 17, wobei die Entzündungskrankheit aus der Gruppe rheumatoide Arthritis, Schuppenflechte, Kontaktdermatitis und Spät-Typ der Überempfindlichkeitsreaktion stammt.

23. Verwendung von Verbindungen der Formel I gemäß Anspruch 1 und/oder ihrer physiologisch unbedenklichen Salze und Solvate zur
Herstellung eines Arzneimittels zur Behandlung von festen Tumoren, wobei eine therapeutisch wirksame Menge einer Verbindung der Formel I in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

24. Verwendung von Verbindungen der Formel I gemäß Anspruch 1 und/oder ihrer physiologisch unbedenklichen Salze und Solvate zur Herstellung eines Arzneimittels zur Behandlung von festen Tumoren wobei eine therapeutisch wirksame Menge einer Verbindung der Formel I in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

## Claims

1. Compounds of the formula I 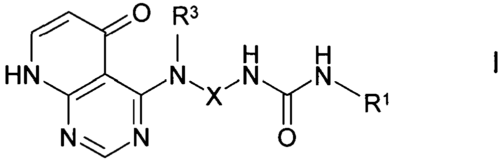 in which
R¹ denotes Ar or Het¹,
R² denotes A, Hal, OH, OA or CN,
R³, R⁴ each, independently of one another, denote H or A,
X denotes phenylene which is unsubstituted or mono-, di-, tri- or tetrasubstituted by R², or a 6-membered aromatic heterocycle having 1-2 N atoms, which is unsubstituted or may be mono-, di-, tri- or tetrasubstituted by R²,
Ar denotes a mono- or bicyclic aromatic carbocycle having 6-10 C atoms, which may be unsubstituted or mono-, di- or trisubstituted by Hal, A, OA, OH, alkenyl having 2 to 6 C atoms, alkynyl having 2 to 6 C atoms, NO₂, NH₂, NHA, NA₂, COOH, COOA, CN, Het, -O-Het, -O-alkylene-Het, -O-alkylene-NR³R⁴, -NR³-alkylene-NR³R⁴, CONR³R⁴ and/or -O-alkylene-NR³-alkylene-OR³,
Het¹ denotes a mono- or bicyclic saturated, unsaturated or aromatic heterocycle having 1 to 4 N, O and/or S atoms, which may be unsubstituted or mono-, di- or trisubstituted by Hal, A, OA, OH, alkenyl having 2 to 6 C atoms, alkynyl having 2 to 6 C atoms, NO₂, NH₂, NHA, NA₂, COOH, COOA, CN, -O-Het, -O-alkylene-Het, -O- alkylene-NR³R⁴, -NR³-alkylene-NR³R⁴, CONR³R⁴ and/or -O-alkylene-NR³-alkylene-OR³,
Het denotes a mono- or bicyclic saturated, unsaturated or aromatic heterocycle having 1 to 4 N, O and/or S atoms, which may be unsubstituted or mono-, di- or trisubstituted by Hal, A, OA, COOA, CN and/or carbonyl oxygen (=O),
A denotes alkyl having 1 to 10 C atoms, in which, in addition, 1-7 H atoms may be replaced by F and/or chlorine,
Hal denotes F, Cl, Br or I,
and pharmaceutically usable derivatives, solvates, salts, tautomers and stereoisomers thereof, including mixtures thereof in all ratios.

2. Compounds according to Claim 1 in which
X denotes phenylene, pyridinediyl or pyrimidinediyl,
and pharmaceutically usable derivatives, solvates, salts, tautomers and stereoisomers thereof, including mixtures thereof in all ratios.

3. Compounds according to Claim 1 or 2 in which
Ar denotes a mono- or bicyclic aromatic carbocycle having 6-10 C atoms, which may be unsubstituted or mono-, di- or trisubstituted by Hal, A, OA, OH, Het, -O-Het, -O-alkylene-Het, -O-alkylene-NR³R⁴, -NR³-alkylene-NR³R⁴, CONR3^{R}4 and/or -O-alkylene-NR³-alkylene-OR³,
and pharmaceutically usable derivatives, solvates, salts, tautomers and stereoisomers thereof, including mixtures thereof in all ratios.

4. Compounds according to one or more of Claims 1-3 in which
Ar denotes phenyl which is unsubstituted or mono-, di- or trisubstituted by Hal, A, OA, OH, -O-alkylene-NR³R⁴, - NR³-alkylene-NR³R⁴, Het, -O-Het and/or -O-alkylene-Het,
and pharmaceutically usable derivatives, solvates, salts, tautomers and stereoisomers thereof, including mixtures thereof in all ratios.

5. Compounds according to one or more of Claims 1-4 in which
Het¹ denotes a monocyclic saturated, unsaturated or aromatic heterocycle having 1 to 3 N, O and/or S atoms, which is unsubstituted or may be mono- or disubstituted by Hal, A, OA and/or OH,
and pharmaceutically usable derivatives, solvates, salts, tautomers and stereoisomers thereof, including mixtures thereof in all ratios.

6. Compounds according to one or more of Claims 1-5 in which
Het¹ denotes a monocyclic aromatic heterocycle having 1 to 3 N, O and/or S atoms, which is unsubstituted or may be mono- or disubstituted by Hal, A, OA and/or OH,
and pharmaceutically usable derivatives, solvates, salts, tautomers and stereoisomers thereof, including mixtures thereof in all ratios.

7. Compounds according to one or more of Claims 1-6 in which
Het¹ denotes a 5- or 6-membered aromatic heterocycle having 1 to 2 N and/or O atoms, which is unsubstituted or may be mono- or disubstituted by A,
and pharmaceutically usable derivatives, solvates, salts, tautomers and stereoisomers thereof, including mixtures thereof in all ratios.

8. Compounds according to one or more of Claims 1-7 in which
Het¹ denotes pyridyl, isoxazolyl, quinolyl, isoquinolyl, thiazolyl, 1,3,4-thiadiazolyl, 1,2,4-thiadiazolyl, furyl, thienyl, pyrrolyl, pyrimidinyl, imidazolyl, pyrazolyl, oxazolyl, isothiazolyl or pyrazinyl, each of which is unsubstituted or mono- or disubstituted by A,
and pharmaceutically usable derivatives, solvates, salts, tautomers and stereoisomers thereof, including mixtures thereof in all ratios.

9. Compounds according to one or more of Claims 1-8 in which
Het denotes a monocyclic saturated, unsaturated or aromatic heterocycle having 1 to 3 N, O and/or S atoms, which is unsubstituted or may be monosubstituted by A,
and pharmaceutically usable derivatives, solvates, salts, tautomers and stereoisomers thereof, including mixtures thereof in all ratios.

10. Compounds according to one or more of Claims 1-9 in which
Het denotes piperidinyl, pyrrolidinyl, morpholinyl, piperazinyl, triazolyl, pyridyl, isoxazolyl, quinolyl, isoquinolyl, thiazolyl, 1,3,4-thiadiazolyl, 1,2,4-thiadiazolyl, furyl, thienyl, pyrrolyl, pyrimidinyl, imidazolyl, pyrazolyl, oxazolyl, isothiazolyl or pyrazinyl, each of which is unsubstituted or monosubstituted by A,
and pharmaceutically usable derivatives, solvates, salts, tautomers and stereoisomers thereof, including mixtures thereof in all ratios.

11. Compounds according to one or more of Claims 1-10 in which
R³, R⁴ denote H,
and pharmaceutically usable derivatives, solvates, salts, tautomers and stereoisomers thereof, including mixtures thereof in all ratios.

12. Compounds according to one or more of Claims 1-11 in which
R¹ denotes Ar or Het¹,
X denotes phenylene, pyridinediyl or pyrimidinediyl,
Ar denotes a mono- or bicyclic aromatic carbocycle having 6-10 C atoms, which may be unsubstituted or mono-, di- or trisubstituted by Hal, A, OA, OH, Het, -O-Het, -O- alkylene-Het, -O-alkylene-NR³R⁴, -NR³-alkylene-NR³R⁴, CONR³R⁴ and/or -O-alkylene-NR³-alkylene-OR³,
Het¹ denotes a monocyclic saturated, unsaturated or aromatic heterocycle having 1 to 3 N, O and/or S atoms, which is unsubstituted or may be mono- or disubstituted by Hal, A, OA and/or OH,
Het denotes a monocyclic saturated, unsaturated or aromatic heterocycle having 1 to 3 N, O and/or S atoms, which is unsubstituted or may be monosubstituted by A,
A denotes alkyl having 1 to 10 C atoms, in which, in addition, 1-7 H atoms may be replaced by F and/or chlorine,
Hal denotes F, Cl, Br or I,
and pharmaceutically usable derivatives, solvates, salts, tautomers and stereoisomers thereof, including mixtures thereof in all ratios.

13. Compounds according to one or more of Claims 1-12 in which
R¹ denotes Ar or Het¹,
X denotes phenylene, pyridinediyl or pyrimidinediyl,
Ar denotes phenyl which is unsubstituted or mono-, di- or trisubstituted by Hal, A, OA, OH, -O-alkylene-NR³R⁴, -NR³-alkylene-NR³R⁴, Het, -O-Het and/or -O-alkylene-Het,
Het¹ denotes a 5- or 6-membered aromatic heterocycle having 1 to 2 N and/or O atoms, which is unsubstituted or may be mono- or disubstituted by A,
Het denotes a monocyclic saturated, unsaturated or aromatic heterocycle having 1 to 3 N, O and/or S atoms, which is unsubstituted or may be monosubstituted by A,
A denotes alkyl having 1 to 10 C atoms, in which, in addition, 1-7 H atoms may be replaced by F and/or chlorine,
Hal denotes F, Cl, Br or I,
and pharmaceutically usable derivatives, solvates, salts, tautomers and stereoisomers thereof, including mixtures thereof in all ratios.

14. Compounds according to Claim 1, selected from the group
| | |
|---|---|
| "1" | 1-(2-Fluoro-5-trifluoromethylphenyl)-3-[4-(5-oxo-5,8-dihydropyrido[2,3-*d*]pyrimidin-4-ylamino)phenyl]urea |
| "2" | 1-(3-Trifluoromethylphenyl)-3-[4-(5-oxo-5,8-dihydropyrido-[2,3-d]pyrimidin-4-ylamino)phenyl]urea |
| "3" | 1-(4-Chloro-5-triftuoromethylphenyl)-3-[4-(5-oxo-5,8-dihydropyrido[2,3-*d*]pyrimidin-4-ylamino)phenyl]urea |
| "4" | 1-(4-Methoxy-5-trifluoromethylphenyl)-3-(4-(5-oxo-5,8-dihydropyrido[2,3-*d*]pyrimidin-4-ylamino)phenyl]urea |
| "5" | 1-(3-Isopropylphenyl)-3-[4-(5-oxo-5,8-dihydropyrido-[2,3-*d*]pyrimidin-4-ylamino)phenyl]urea |
| "6" | 1-(2-Methoxy-5-trifluoromethylphenyl)-3-[4-(5-oxo-5,8-dihydropyrido[2,3-*d*]pyrimidin-4-ylamino)phenyl]urea |
| "7" | 1-(3-Fluoro-5-trifluoromethylphenyl)-3-[4-(5-oxo-5,8-dihydropyrido[2,3-*d*]pyrimidin-4-ylamino)phenyl]urea |
| "8" | 1-(4-Fluoro-5-trifluoromethylphenyl)-3-[4-(5-oxo-5,8-dihydropyrido[2,3-d]pyrimidin-4-ylamino)phenyl]urea |
| "9" | 1-(3-Trifluoromethoxyphenyl)-3-[4-(5-oxo-5,8-dihydro-pyrido[2,3-*d*]pyrimidin-4-ylamino)phenyl]urea |
| "10" | 1-[2-(3-Morpholin-4-ylpropoxy)-5-trifluoromethylphenyl]-3-[4-(5-oxo-5,8-dihydropyrido[2,3-*d*]pyrimidin-4-ylamino)-phenyl]urea |
| "11" | 1-[4-(3-Morpholin-4-ylpropoxy)-5-trifluoromethylphenyl]-3-[4-(5-oxo-5,8-dihydropyrido[2,3-*d*]pyrimidin-4-ylamino)-phenyl]urea |
| "12" | 1-(3-Isopropoxyphenyl)-3-[4-(5-oxo-5,8-dihydropyrido-[2,3-*d*]pyrimidin-4-ylamino)phenyl]urea |
| "13" | 1-(3-Trifluoromethylphenyl)-3-[2-(5-oxo-5,8-dihydropyrido-[2,3-*d*]pyrimidin-4-ylamino)pyridin-5-yl]urea |
| "14" | 1-(3-Trifluoromethylphenyl)-3-[4-(5-oxo-5,8-dihydropyrido-[2,3-*d*]pyrimidin-4-ylamino)phenyl]urea |
| "15" | 1-[2-(1,2,4-Triazo)-1-yl)-5-thifluoromethy)pheny)]-3-[4-(5-oxo-5,8-dihydropyrido[2,3-*d*]pyrimidin-4-ylamino)phenyl]-urea |
| "16" | 1-(5-tert-Butylisoxazol-3-yl)-3-[4-(5-oxo-5,8-dihydropyrido-[2,3-*d*]pyrimidin-4-ylamino)phenyl]urea |
| "17" | 1-(3-Trifluoromethylphenyl)-3-[2-(5-oxo-5,8-dihydropyrido-[2,3-*d*]pyrimidin-4-ylamino)pyrimidin-5-yl]urea |
| "18" | 1-(3-Ethylphenyl)-3-[4-(5-oxo-5,8-dihydropyrido[2,3-*d*]-pyrimidin-4-ylamino)phenyl]urea |
| "19" | 1-[6-(1-Methylpiperidin-4-yloxy)-3-trifluoromethylphenyl]-3-[2-(5-oxo-5,8-dihydropyrido[2,3-*d*]pyrimidin-4-ylamino)-pyridin-5-yl]urea |
| "20" | 1-[6-(1-Methylpiperidin-4-ylmethoxy)-3-trifluoromethyl-phenyl]-3-[2-(5-oxo-5,8-dihydropyrido[2,3-*d*]pyrimidin-4-ylamino)pyridin-5-yl]urea |
| "21" | 1-(3-Trifluoromethylphenyl)-3-[3-(5-oxo-5,8-dihydropyrido-[2,3-*d*]pyrimidin-4-ylamino)phenyl]urea |
| "22" | 1-(3-Isopropylphenyl)-3-[3-(5-oxo-5,8-dihydropyrido-[2,3-d]pyrimidin-4-ylamino)phenyl]urea |
| "23" | 1-(3-Trifluoromethoxyphenyl)-3-[3-(5-oxo-5,8-dihydro-pyrido[2,3-*d*]pyrimidin-4-ylamino)phenyl]urea |
| "24" | 1-[4-(1-Methylpiperidin-4-yloxy)-3-trifluoromethylphenyl]-3-[4-(5-oxo-5,8-dihydropyrido[2,3-*d*]pyrimidin-4-ylamino)-phenyl]urea |
| "26" | 1-[4-(2-Methylaminoethoxy)-3-trifluoromethylphenyl]-3-[4-(5-oxo-5,8-dihydropyrido[2,3-d]pyrimidin-4-ylamino)-phenyl]urea |
and pharmaceutically usable derivatives, solvates, salts, tautomers and stereoisomers thereof, including mixtures thereof in all ratios.

15. Process for the preparation of compounds of the formula I according to Claims 1-14 and pharmaceutically usable derivatives, salts, solvates, tautomers and stereoisomers thereof, **characterised in that**
a) a compound of the formula II 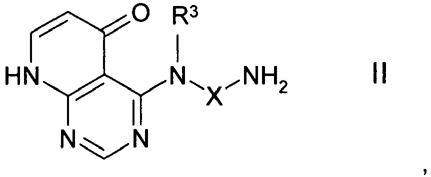 in which X and R³ have the meanings indicated in Claim 1,
is reacted with a compound of the formula III
R¹-N=C=O III ,
in which R¹ has the meaning indicated in Claim 1,
or
b) a compound of the formula II is reacted with a compound of the formula IV
R¹-NH₂ IV ,
in which R¹ has the meaning indicated in Claim 1,
and a chlorocarbonic acid ester,
and/or
a base or acid of the formula I is converted into one of its salts.

16. Medicaments comprising at least one compound of the formula I according to Claim 1 and/or pharmaceutically usable derivatives, salts, solvates, tautomers and stereoisomers thereof, including mixtures thereof in all ratios, and optionally excipients and/or adjuvants.

17. Use of compounds according to Claim 1
and pharmaceutically usable derivatives, salts, solvates, tautomers and stereoisomers thereof, including mixtures thereof in all ratios, for the preparation of a medicament for the treatment of diseases in which the inhibition, regulation and/or modulation of kinase signal transduction plays a role, where the diseases are selected from the group
solid tumour, tumour of the blood and immune system, eye disease, retinal vascularisation, diabetic retinopathy, age-induced macular degeneration, inflammatory diseases, bone pathologies, where the bone pathology originates from the group osteosarcoma, osteoarthritis and rickets, psoriasis, arthritis, inflammations, endometriosis, scarring, benign prostate hyperplasia, immunological diseases, autoimmune diseases, immunodeficiency diseases.

18. Use according to Claim 17, where the solid tumour originates from the group of tumours of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx and/or of the lung.

19. Use according to Claim 17, where the solid tumour originates from the group monocytic leukaemia, lung adenocarcinoma, small-cell lung carcinomas, pancreatic cancer, glioblastomas and breast carcinoma.

20. Use according to Claim 17, where the solid tumour originates from the group of lung adenocarcinoma, small-cell lung carcinomas, pancreatic cancer, glioblastomas, colon carcinoma and breast carcinoma.

21. Use according to Claim 17, where the tumour of the blood and immune system originates from the group of acute myeloid leukaemia, chronic myeloid leukaemia, acute lymphatic leukaemia and/or chronic lymphatic leukaemia.

22. Use according to Claim 17, where the inflammatory disease originates from the group rheumatoid arthritis, psoriasis, contact dermatitis and delayed-type hypersensitivity reaction.

23. Use of compounds of the formula I according to Claim 1 and/or physiologically acceptable salts and solvates thereof for the preparation of a medicament for the treatment of solid tumours, where a therapeutically effective amount of a compound of the formula I is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitor.

24. Use of compounds of the formula I according to Claim 1 and/or physiologically acceptable salts and solvates thereof for the preparation of a medicament for the treatment of solid tumours, where a therapeutically effective amount of a compound of the formula I is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitor.

## Revendications

1. Composés de formule I 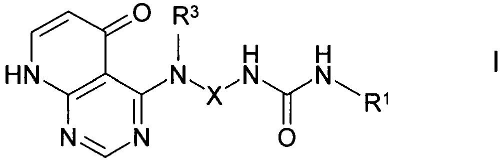 dans laquelle
R¹ désigne Ar ou Hét¹,
R² désigne A, Hal, OH, OA ou CN,
R ³ R⁴ désignent chacun, indépendamment l'un de l'autre, H ou A,
X désigne phénylène qui est non substitué ou mono-, di-, tri- ou tétrasubstitué par R² ou un hétérocycle aromatique de 6 chaînons ayant 1-2 atomes de N, qui est non substitué ou peut être mono-, di-, tri- ou tétrasubstitué par R²
Ar désigne un carbocycle aromatique mono- ou bicyclique ayant 6-10 atomes de C, pouvant être non substitué ou mono-, di- ou trisubstitué par Hal, A, OA, OH, alcényle ayant 2 à 6 atomes de C, alcynyle ayant 2 à 6 atomes de C, NO₂, NH₂, NHA, NA₂, COOH, COOA, CN, Hét, -O-Hét, -O-alkylène-Hét, -O-alkylène-NR³R⁴, -NR³-alkylène-NR³R⁴, CONR³R⁴ et/ou -O-alkylène-NR³-alkylène-OR³
Hét¹ désigne un hétérocycle mono- ou bicyclique saturé, insaturé ou aromatique ayant 1 à 4 atomes de N, O et/ou S, pouvant être non substitué ou mono-, di- ou trisubstitué par Hal, A, OA, OH, alcényle ayant 2 à 6 atomes de C, alcynyle ayant 2 à 6 atomes de C, NO₂, NH₂, NHA, NA₂, COOH, COOA, CN, -O-Hét, -O-alkylène-Hét, -O-alkylène-NR³R⁴, -NR³-alkylène-NR³R⁴, CONR³R⁴ et/ou -O-alkylène-NR³-alkylène-OR³,
Hét désigne un hétérocycle mono- ou bicyclique saturé, insaturé ou aromatique ayant 1 à 4 atomes de N, O et/ou S, pouvant être non substitué ou mono-, di- ou trisubstitué par Hal, A, OA, COOA, CN et/ou oxygène de carbonyle (=O),
A désigne alkyle ayant 1 à 10 atomes de C, dans lequel, en outre, 1-7 atomes de H peuvent être remplacés par F et/ou chlore,
Hal désigne F, CI, Br ou I,
et des dérivés, solvats, sels, tautomères et stéréoisomères pharmaceutiquement utilisables de ceux-ci, y compris des mélanges de ceux-ci en toutes proportions.

2. Composés selon la revendication 1, dans lesquels
X désigne phénylène, pyridinediyle ou pyrimidinediyle,
et des dérivés, solvats, sels, tautomères et stéréoisomères pharmaceutiquement utilisables de ceux-ci, y compris des mélanges de ceux-ci en toutes proportions.

3. Composés selon la revendication 1 ou 2, dans lesquels
Ar désigne un carbocycle aromatique mono- ou bicyclique ayant 6-10 atomes de C, pouvant être non substitué ou mono-, di- ou trisubstitué par Hal, A, OA, OH, Hét, -O-Hét, -O-alkylène-Hét, -O-alkylène-NR³R⁴, -NR³-alkylène-NR³R⁴, CONR³R⁴ et/ou -O-alkylène-NR³-alkylène-OR³,
et des dérivés, solvats, sels, tautomères et stéréoisomères pharmaceutiquement utilisables de ceux-ci, y compris des mélanges de ceux-ci en toutes proportions.

4. Composés selon l'une ou plusieurs des revendications 1-3, dans lesquels
Ar désigne phényle qui est non substitué ou mono-, di- ou trisubstitué par Hal, A, OA, OH, -O-alkylène-NR³R⁴, -NR³-alkylène-NR³R⁴, Hét, -O-Hét et/ou -O-alkylène-Hét,
et des dérivés, solvats, sels, tautomères et stéréoisomères pharmaceutiquement utilisables de ceux-ci, y compris des mélanges de ceux-ci en toutes proportions.

5. Composés selon l'une ou plusieurs des revendications 1-4, dans lesquels
Hét¹ désigne un hétérocycle monocyclique saturé, insaturé ou aromatique ayant 1 à 3 atomes de N, O et/ou S, qui est non substitué ou peut être mono- ou disubstitué par Hal, A, OA et/ou OH,
et des dérivés, solvats, sels, tautomères et stéréoisomères pharmaceutiquement utilisables de ceux-ci, y compris des mélanges de ceux-ci en toutes proportions.

6. Composés selon l'une ou plusieurs des revendications 1-5, dans lesquels
Hét¹ désigne un hétérocycle monocyclique aromatique ayant 1 à 3 atomes de N, O et/ou S, qui est non substitué ou peut être mono- ou disubstitué par Hal, A, OA et/ou OH,
et des dérivés, solvats, sels, tautomères et stéréoisomères pharmaceutiquement utilisables de ceux-ci, y compris des mélanges de ceux-ci en toutes proportions.

7. Composés selon l'une ou plusieurs des revendications 1-6, dans lesquels
Hét¹ désigne un hétérocycle aromatique de 5 ou 6 chaînons ayant 1 à 2 atomes de N et/ou O, qui est non substitué ou peut être mono- ou disubstitué par A,
et des dérivés, solvats, sels, tautomères et stéréoisomères pharmaceutiquement utilisables de ceux-ci, y compris des mélanges de ceux-ci en toutes proportions.

8. Composés selon l'une ou plusieurs des revendications 1-7, dans lesquels
Hét¹ désigne pyridyle, isoxazolyle, quinolyle, isoquinolyle, thiazolyle, 1,3,4-thiadiazolyle, 1,2,4-thiadiazolyle, furyle, thiényle, pyrrolyle, pyrimidinyle, imidazolyle, pyrazolyle, oxazolyle, isothiazolyle ou pyrazinyle, chacun d'entre eux étant non substitué ou mono- ou disubstitué par A,
et des dérivés, solvats, sels, tautomères et stéréoisomères pharmaceutiquement utilisables de ceux-ci, y compris des mélanges de ceux-ci en toutes proportions.

9. Composés selon l'une ou plusieurs des revendications 1-8, dans lesquels
Hét désigne un hétérocycle monocyclique saturé, insaturé ou aromatique ayant 1 à 3 atomes de N, O et/ou S, qui est non substitué ou peut être monosubstitué par A,
et des dérivés, solvats, sels, tautomères et stéréoisomères pharmaceutiquement utilisables de ceux-ci, y compris des mélanges de ceux-ci en toutes proportions.

10. Composés selon l'une ou plusieurs des revendications 1-9, dans lesquels
Hét désigne pipéridinyle, pyrrolidinyle, morpholinyle, pipérazinyle, triazolyle, pyridyle, isoxazolyle, quinolyle, isoquinolyle, thiazolyle, 1,3,4-thiadiazolyle, 1,2,4-thia- diazolyle, furyle, thiényle, pyrrolyle, pyrimidinyle, imidazolyle, pyrazolyle, oxazolyle, isothiazolyle ou pyrazinyle, chacun d'entre eux étant non substitué ou monosubstitué par A,
et des dérivés, solvats, sels, tautomères et stéréoisomères pharmaceutiquement utilisables de ceux-ci, y compris des mélanges de ceux-ci en toutes proportions.

11. Composés selon l'une ou plusieurs des revendications 1-10, dans lesquels
R³, R⁴ désignent H,
et des dérivés, solvats, sels, tautomères et stéréoisomères pharmaceutiquement utilisables de ceux-ci, y compris des mélanges de ceux-ci en toutes proportions.

12. Composés selon l'une ou plusieurs des revendications 1-11, dans lesquels
R¹ désigne Ar ou Hét¹,
X désigne phénylène, pyridinediyle ou pyrimidinediyle,
Ar désigne un carbocycle aromatique mono- ou bicyclique ayant 6-10 atomes de C, pouvant être non substitué ou mono-, di- ou trisubstitué par Hal, A, OA, OH, Hét, -O-Hét, -O-alkylène-Hét, -O-alkylène-NR³R⁴, -NR³-alkylène-NR³R⁴, CONR³R⁴ et/ou -O-alkylène-NR³-alkylène-OR³,
Hét¹ désigne un hétérocycle monocyclique saturé, insaturé ou aromatique ayant 1 à 3 atomes de N, O et/ou S, qui est non substitué ou peut être mono- ou disubstitué par Hal, A, OA et/ou OH,
Hét désigne un hétérocycle monocyclique saturé, insaturé ou aromatique ayant 1 à 3 atomes de N, O et/ou S, qui est non substitué ou peut être monosubstitué par A,
A désigne alkyle ayant 1 à 10 atomes de C, dans lequel, en outre, 1-7 atomes de H peuvent être remplacés par F et/ou chlore,
Hal désigne F, Cl, Br ou I,
et des dérivés, solvats, sels, tautomères et stéréoisomères pharmaceutiquement utilisables de ceux-ci, y compris des mélanges de ceux-ci en toutes proportions.

13. Composés selon l'une ou plusieurs des revendications 1-12, dans lesquels
R¹ désigne Ar ou Hét¹,
X désigne phénylène, pyridinediyle ou pyrimidinediyle,
Ar désigne phényle qui est non substitué ou mono-, di- ou trisubstitué par Hal, A, OA, OH, -O-alkylène-NR³R⁴, -NR³-alkylène-NR³R⁴, Hét, -O-Hét et/ou -O-alkylène-Hét,
Hét¹ désigne un hétérocycle aromatique de 5 ou 6 chaînons ayant 1 à 2 atomes de N et/ou O, qui est non substitué ou peut être mono- ou disubstitué par A,
Hét désigne un hétérocycle monocyclique saturé, insaturé ou aromatique ayant 1 à 3 atomes de N, O et/ou S, qui est non substitué ou peut être monosubstitué par A,
A désigne alkyle ayant 1 à 10 atomes de C, dans lequel, en outre, 1-7 atomes de H peuvent être remplacés par F et/ou chlore,
Hal désigne F, Cl, Br ou I,
et des dérivés, solvats, sels, tautomères et stéréoisomères pharmaceutiquement utilisables de ceux-ci, y compris des mélanges de ceux-ci en toutes proportions.

14. Composés selon la revendication 1, choisis dans le groupe constitué par :
| | |
|---|---|
| "1" | 1-(2-Fluoro-5-trifluorométhylphényl)-3-[4-(5-oxo-5,8-dihydropyrido[2,3-*d*]pyrimidin-4-ylamino)phényl]urée |
| "2" | 1-(3-Trifluorométhylphényl)-3-[4-(5-oxo-5,8-dihydropyrido-[2,3-*d*]pyrimidin-4-ylamino)phényl]urée |
| "3" | 1-(4-Chloro-5-trifluorométhylphényl)-3-[4-(5-oxo-5,8-dihydropyrido[2,3-*d*]pyrimidin-4-ylamino)phényl]urée |
| "4" | 1-(4-Méthoxy-5-trifluorométhylphényl)-3-[4-(5-oxo-5,8-dihydropyrido[2,3-*d*]pyrimidin-4-ylamino)phényl]urée |
| "5" | 1-(3-Isopropylphényl)-3-[4-(5-oxo-5,8-dihydropyrido-[2,3-*d*]pyrimidin-4-ylamino)phényl]urée |
| "6" | 1-(2-Méthoxy-5-trifluorométhylphényl)-3-[4-(5-oxo-5,8-dihydropyrido[2,3-d]pyrimidin-4-ylamino)phényl]urée |
| "7" | 1-(3-Fluoro-5-trifluorométhylphényl)-3-[4-(5-oxo-5,8-dihydropyrido[2,3-*d*]pyrimidin-4-ylamino)phényl]urée |
| "8" | 1-(4-Fluoro-5-trifluorométhylphényl)-3-[4-(5-oxo-5,8-dihydropyrido[2,3-*d*]pyrimidin-4-ylamino)phényl]urée |
| "9" | 1-(3-Trifluorométhoxyphényl)-3-[4-(5-oxo-5,8-dihydro-pyrido[2,3-d]pyrimidin-4-ylamino)phényl]urée |
| "10" | 1-[2-(3-Morpholin-4-ylpropoxy)-5-trifluorométhylphényl]-3-[4-(5-oxo-5,8-dihydropyrido[2,3-*d*]pyrimidin-4-ylamino)-phényl]urée |
| "11" | 1-[4-(3-Morpholin-4-ylpropoxy)-5-trifluorométhylphényl]-3-[4-(5-oxo-5,8-dihydropyrido[2,3-*d*]pyrimidin-4-ylamino)-phényl]urée |
| "12" | 1-(3-Isopropoxyphényl)-3-[4-(5-oxo-5,8-dihydropyrido-[2,3-*d*]pyrimidin-4-ylamino)phényl]urée |
| "13" | 1-(3-Trifluorométhylphényl)-3-[2-(5-oxo-5,8-dihydropyrido-[2,3-*d*]pyrimidin-4-ylamino)pyridin-5-yl]urée |
| "14" | 1-(3-Trifluorométhylphényl)-3-[4-(5-oxo-5,8-dihydropyrido-[2,3-*d*]pyrimidin-4-ylamino)phényl]urée |
| "15" | 1-[2-(1,2,4-Triazol-1-yl)-5-trifluorométhylphényl]-3-[4-(5-oxo-5,8-dihydropyrido[2,3-*d*]pyrimidin-4-ylamino)phényl]-urée |
| "16" | 1-(5-tertio-Butylisoxazol-3-yl)-3-[4-(5-oxo-5,8-dihydro-pyrido[2,3-*d*]pyrimidin-4-ylamino)phényl]urée |
| "17" | 1-(3-Trifluorométhylphényl)-3-[2-(5-oxo-5,8-dihydropyrido-[2,3-*d*]pyrimidin-4-ylamino)pyrimidin-5-yl]urée |
| "18" | 1-(3-Ethylphényl)-3-[4-(5-oxo-5,8-dihydropyrido[2,3-*d*]-pyrimidin-4-ylamino)phényl]urée |
| "19" | 1-[6-(1-Méthylpipéridin-4-yloxy)-3-trifluorométhylphényl]-3-[2-(5-oxo-5,8-dihydropyrido[2,3-*d*]pyrimidin-4-ylamino)-pyridin-5-yl]urée |
| "20" | 1-[6-(1-Méthylpipéridin-4-ylméthoxy)-3-trifluorométhyl-phényl]-3-[2-(5-oxo-5,8-dihydropyrido[2,3-*d*]pyrimidin-4-ylamino)pyridin-5-yl]urée |
| "21" | 1-(3-Trifluorométhylphényl)-3-[3-(5-oxo-5,8-dihydropyrido-[2,3-*d*]pyrimidin-4-ylamino)phényl]urée |
| "22" | 1-(3-Isopropylphényl)-3-[3-(5-oxo-5,8-dihydropyrido-[2,3-*d*]pyrimidin-4-ylamino)phényl]urée |
| "23" | 1-(3-Trifluorométhoxyphényl)-3-[3-(5-oxo-5,8-dihydro-pyrido[2,3-*d*]pyrimidin-4-ylamino)phényl]urée |
| "24" | 1-[4-(1-Méthylpipérid in-4-yloxy)-3-trifluorométhylphényl]-3-[4-(5-oxo-5,8-dihydropyrido[2,3-*d*]pyrimidin-4-ylamino)-phényl]urée |
| "26" | 1-[4-(2-Méthylaminoéthoxy)-3-trifluorométhylphényl]-3-[4-(5-oxo-5,8-dihydropyrido[2,3-*d*]pyrimidin-4-ylamino)-phényl]urée |
et des dérivés, solvats, sels, tautomères et stéréoisomères pharmaceutiquement utilisables de ceux-ci, y compris des mélanges de ceux-ci en toutes proportions.

15. Procédé de préparation de composés de formule I selon les revendications 1-14 et de dérivés, sels, solvats, tautomères et stéréoisomères pharmaceutiquement utilisables de ceux-ci, **caractérisé en ce que**
a) un composé de formule II 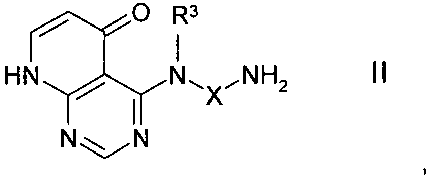 dans laquelle X et R³ ont les significations indiquées selon la revendication 1,
est réagi avec un composé de formule III
R¹-N=C=O III ,
dans laquelle R¹ a la signification indiquée selon la revendication 1,
ou
b) un composé de formule II est réagi avec un composé de formule IV
R¹-NH₂ IV ,
dans laquelle R¹ a la signification indiquée selon la revendication 1,
et un ester d'acide chlorocarbonique,
et/ou
une base ou un acide de formule I est converti(e) en l'un de ses sels.

16. Médicaments comprenant au moins un composé de formule I selon la revendication 1 et/ou des dérivés, sels, solvats, tautomères et stéréoisomères pharmaceutiquement utilisables de celui-ci, y compris des mélanges de ceux-ci en toutes proportions, et éventuellement des excipients et/ou des adjuvants.

17. Utilisation de composés selon la revendication 1
et de dérivés, sels, solvats, tautomères et stéréoisomères pharmaceutiquement utilisables de ceux-ci, y compris des mélanges de ceux-ci en toutes proportions,
pour la préparation d'un médicament destiné au traitement de maladies dans lesquelles l'inhibition, la régulation et/ou la modulation de la transduction des signaux de kinases joue un rôle, où les maladies sont sélectionnées dans le groupe constitué par :
les tumeurs solides, les tumeurs du système sanguin et immunitaire, les maladies oculaires, la vascularisation rétinienne, la rétinopathie diabétique, la dégénérescence maculaire liée à l'âge, les maladies inflammatoires, les pathologies osseuses, où la pathologie osseuse provient du groupe constitué par l'ostéosarcome, l'arthrose et le rachitisme, le psoriasis, l'arthrite, les inflammations, l'endométriose, la cicatrisation, l'hyperplasie bénigne de la prostate, les maladies immunologiques, les maladies auto-immunes et les maladies d'immunodéficience.

18. Utilisation selon la revendication 17, dans laquelle la tumeur solide provient du groupe constitué par les tumeurs de l'épithélium squameux, de la vessie, de l'estomac, des reins, de la tête et du cou, de l'oesophage, du col de l'utérus, de la thyroïde, des intestins, du foie, du cerveau, de la prostate, du tractus urogénital, du système lymphatique, de l'estomac, du larynx et/ou du poumon.

19. Utilisation selon la revendication 17, dans laquelle la tumeur solide provient du groupe constitué par la leucémie monocytique, l'adénocarcinome du poumon, les carcinomes du poumon à petites cellules, le cancer du pancréas, les glioblastomes et le carcinome du sein.

20. Utilisation selon la revendication 17, dans laquelle la tumeur solide provient du groupe constitué par l'adénocarcinome du poumon, les carcinomes du poumon à petites cellules, le cancer du pancréas, les glioblastomes, le carcinome du côlon et le carcinome du sein.

21. Utilisation selon la revendication 17, dans laquelle la tumeur du système sanguin et immunitaire provient du groupe constitué par la leucémie myéloïde aiguë, la leucémie myéloïde chronique, la leucémie lymphatique aiguë et/ou la leucémie lymphatique chronique.

22. Utilisation selon la revendication 17, dans laquelle la maladie inflammatoire provient du groupe constitué par la polyarthrite rhumatoïde, le psoriasis, la dermite de contact et la réaction d'hypersensibilité retardée.

23. Utilisation de composés de formule I selon la revendication 1 et/ou de sels et de solvats physiologiquement acceptables de ceux-ci, pour la préparation d'un médicament destiné au traitement de tumeurs solides, dans laquelle une quantité thérapeutiquement efficace d'un composé de formule I est administrée en association avec un composé issu du groupe 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) un autre inhibiteur de l'angiogenèse.

24. Utilisation de composés de formule I selon la revendication 1 et/ou de sels et de solvats physiologiquement acceptables de ceux-ci, pour la préparation d'un médicament destiné au traitement de tumeurs solides, dans laquelle une quantité thérapeutiquement efficace d'un composé de formule I est administrée en association avec une radiothérapie et un composé issu du groupe 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) un autre inhibiteur de l'angiogenèse.
